(19) Europäisches Patentamt
European Patent Office
Office européen des brevets

(11) Numéro de publication: 0 053 541
B1

(12) # FASCICULE DE BREVET EUROPEEN

(45) Date de publication du fascicule du brevet: **13.02.85**

(21) Numéro de dépôt: **81401829.7**

(22) Date de dépôt: **19.11.81**

(51) Int. Cl.⁴: **C 07 D 498/04** //
(C07D498/04, 265/00, 205/00)

(54) **Nouvelles oxacéphalosporines et leur préparation.**

(30) Priorité: **20.11.80 FR 8024639**

(43) Date de publication de la demande:
**09.06.82 Bulletin 82/23**

(45) Mention de la délivrance du brevet:
**13.02.85 Bulletin 85/07**

(84) Etats contractants désignés:
**AT BE CH DE FR GB IT LI LU NL SE**

(56) Documents cités:
**FR-A-2 385 722**
**FR-A-2 457 296**

(73) Titulaire: **RHONE-POULENC SANTE**
**Les Miroirs 18 Avenue d'Alsace**
**F-92400 Courbevoie Cedex (FR)**

(72) Inventeur: **Farge, Daniel**
**30 rue des Pins Sylvestres**
**F-94320 Thiais (FR)**
Inventeur: **Le Roy, Pierre**
**2 allée des Cerisiers**
**F-94320 Thiais (FR)**
Inventeur: **Moutonnier, Claude**
**3 rue Auguste Rodin**
**F-92350 Le Plessis-Robinson (FR)**
Inventeur: **Peyronel, Jean-François**
**36 parc d'Ardenay**
**F-91110 Palaiseau (FR)**
Inventeur: **Plau, Bernard**
**53 rue de Mesly**
**F-94000 Créteil (FR)**

(74) Mandataire: **Gaumont, Robert et al**
**RHONE-POULENC RECHERCHES Service**
**Brevets Pharma 25, Quai Paul Doumer**
**F-92408 Courbevoie Cedex (FR)**

EP 0 053 541 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

Dans la demande française FR-A- 2 385 722 ont été décrits des dérivés de céphalosporines et oxacéphalosporines de formule générale:

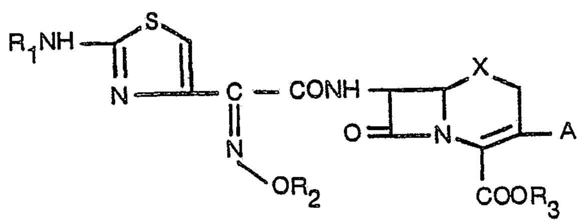

dans laquelle X est notamment un atome de soufre ou d'xygène et A peut prendre la signification alcényloxy.

Ces produits manifestent une activité antibactérienne.

La présente invention concerne de nouveaux dérivés d'oxacéphalosporines de formule générale:

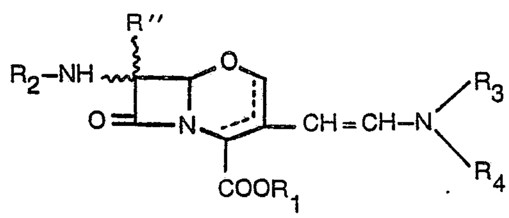

(I)

et leur préparation.

Les produits de formule générale (I) se présentent sous forme bicyclooctène-2 ou -3 (selon la nomenclature des Chemical Abstracts), le substituant sur l'atome de carbone en position -3 du bicyclooctène présente la stéréoisomérie E ou Z et le symbole $R_1$ représente un radical protecteur d'acide facilement éliminable (par exemple méthoxyméthyle, t.butyle, benzhydryle, benzyle, nitrobenzyle ou p.méthoxybenzyle),

a) le symbole $R_2$ représente un radical de formule générale:

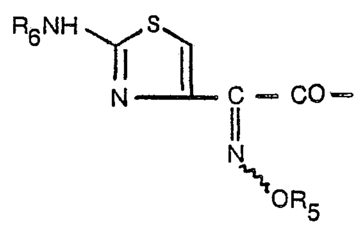

(II)

[dans laquelle $R_5$ est un atome d'hydrogène, un radical alcoyle, vinyle, carboxyalcoyle représenté par la formule générale:

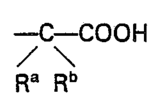

(III)

(dans laquelle $R^a$ et $R^b$ qui sont identiques ou différents représentent des atomes d'hydrogène ou des radicaux alcoyle ou forment ensemble un radical alcoylène contenant 2 ou 3 atomes de carbone) et dont la fonction acide est protégée, ou un radical protecteur d'oxime, et $R_6$ est un radical protecteur d'amine], ou $R_2$ représente un radical α-carboxyarylacétyle dont la fonction acide est protégée (et dans lequel aryle représente phényle, p.hydroxyphényle, p.hydroxyphényle protégé, ou thiényle-2 ou -3), et le symbole R'' représente un atome d'hydrogène ou un radical méthoxy en position 7α, ou bien

b) le symbole $R_2$ représente un radical protecteur d'amine facilement éliminable et le symbole R'' représente un atome d'hydrogène ou un radical méthoxy en 7α ou un atome d'hydrogène en 7β, et les symboles $R_3$ et $R_4$, qui sont identiques ou différents, représentent des radicaux alcoyle (éventuellement

substitués par un radical alcoyloxy ou dialcoylamino) ou phényle, ou forment ensemble avec l'atome d'azote auquel ils sont rattachés un hétérocycle saturé à 5 ou 6 chaînons, contenant éventuellement un autre hétéroatome choisi parmi l'azote, l'oxygène ou le soufre et éventuellement substitué par un radical alcoyle.

Il est entendu que, sauf mention spéciale, les portions ou radicaux alcoyles ou acyles cités ci-dessus (ou qui seront cités ci-après) sont droits ou ramifiés et contiennent 1 à 4 atomes de carbone.

Il est également entendu que le substituant en position (−3) des produits de formule générale (I) peut se présenter sous forme cis ou trans ou d'un mélange des formes cis et trans.

Dans ce qui suit la stéréoisomérie trans sera désignée par E et la stéréoisomérie cis sera désignée par Z.

Par ailleurs, il est entendu que le groupement $OR_5$ du radical de formule générale (II) peut se trouver dans l'une des positions syn ou anti et que ces isomères et leurs mélanges entrent dans le cadre de la présente invention.

La forme syn peut être représentée par la formule:

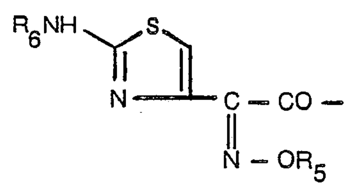

(IIa)

La forme anti peut être représentée par la formule:

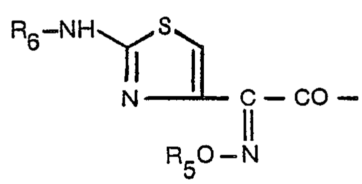

(IIb)

A titre d'exemples,

— les groupements carboxy sont ou peuvent être protégés par des radicaux tels que méthoxyméthyle, t.butyle, benzhydryle, benzyle, nitrobenzyle ou p.méthoxybenzyle.

— les groupements hydroxy, ou l'oxime de $R_2$ lorsque $R_5$ représente un atome d'hydrogène, peuvent être protégés par des radicaux tels que trityle, tétrahydropyrannyle, méthoxy-2 propyle-2, alcoyloxycarbonyle (tel que t.butoxycarbonyle) ou aryloxycarbonyle (tel que benzyloxycarbonyle).

— les groupements protecteurs d'amines représentés par $R_6$ peuvent être des radicaux tel que t-butoxycarbonyle, trichloro-2,2,2 éthoxycarbonyle, trichloracétyle, trityle, benzyle, dibenzyle, benzyloxycarbonyle, p.nitrobenzyloxycarbonyle, p.méthoxybenzyloxycarbonyle, chloracétyle, formyle ou trifluoracétyle.

Par radical $R_2$ facilement éliminable, on entend:

1) benzhydryle ou trityle,

2) un radical acyle de formule générale:

$$R_7CO— \qquad (IV)$$

dans laquelle $R_7$ représente:

a) un atome d'hydrogène, un radical alcoyle contenant 1 à 7 atomes de carbone, méthyle substitué par 1 à 3 atomes d'halogène, alcényle contenant 3 à 7 atomes de carbone ou cyanométhyle,

b) un radicals phényle pouvant être jusqu'à 3 fois substitué (par des radicaux hydroxy, nitro, cyano, trifluorométhyle, alcoyle ou alcoyloxy) ou un radical thiényle-2 ou -3

c) un radical de formule générale:

$$R'_7YCH_2— \qquad (Va)$$

dans laquelle $R'_7$ est un radical tel que défini en b) et Y est un atome de soufre ou d'oxygène

d) un raical arylalcoyl de formule générale:

$$R''_7CH_2— \qquad (Vb)$$

dans laquelle $R_7$ est un radical phényle (pouvant être jusqu'à 3 fois substitué par des radicaux hydroxy, alcoyle ou alcoyloxy) ou un radical hétérocyclyle tel que thiényle-2 ou -3, furyle-2 ou -3 ou tétrazolyle-1

3) un radical de formule générale:

$$R_8OCO— \quad (VI)$$

dans lequelle $R_8$ est un radical alcoyle ramifié non substitué, un radical alcoyle droit ou ramifié portant un ou plusieurs substituants [tels que des atomes d'halogène ou des radicaux cyano, phényle ou phényle substitué (par un ou plusieurs atomes d'halogène ou radicaux alcoyle, alcoyloxy, nitro ou phényle)], un radical triméthylsilyl-2 éthyle, un radical vinyle ou allyle ou un radical quinolyle,

4) un radical de formule générale:

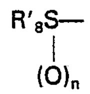 ou $R'_8Se$—

(VII) (VIII)

dans lesquelles le reste $R'_8$ est un radical alcoyle, phényle ou phényle substitué (par un ou plusieurs atomes d'halogène ou radicaux nitro ou alcoyle) et n est égal à 0 ou 1, ou bien

5) $R_2NH$— peut être remplacé par un radical diméthylaminométhylèneamino ou par un radical de formule générale:

$$Ar—CH{=}N— \quad (IX)$$

dans laquelle Ar est un groupe phényle éventuellement substitué par un ou plusieurs radicaux tels que hydroxy, méthoxy, alcoyle ou nitro.

Comme exemples de radicaux $R_2$ pouvant être utilisés, on peut citer les radicaux suivants: formyle, acétyle, chloracétyle, trichloracétyle, phénylacétyle, phénoxyacétyle, benzoyle, t.butoxycarbonyle, chloro-2 diméthyl-1,1 éthoxycarbonyle, trichloro-2,2,2 éthoxycarbonyle, trichloro-2,2,2 diméthyl-1,1 éthoxycarbonyle, cyano-2 diméthyl-1,1 éthoxycarbonyle, triméthylsilyl-2 éthoxycarbonyle, benzyloxycarbonyle, p.méthoxybenzyloxycarbonyle, diméthoxy-3,5 benzyloxycarbonyle, p.nitrobenzyloxycarbonyle, diphénylméthoxycarbonyle, (biphénylyl-4)-2 isopropyloxycarbonyle, vinyloxycarbonyle, allyloxycarbonyle, quinolyl-8 oxycarbonyle, o-nitrophénylthio, p-nitrophénylthio.

Comme exemples de radicaux méthylèneamino définis précédemment en 5°), on peut citer: diméthylaminométhylèneamino, diméthoxy-3,4 benzylidèneamino, nitro-4 benzylidèneamino, di t.butyl-3,5 hydroxy-4 benzylidèneamino.

Selon l'invention, les produits de formule générale (I) peuvent être obtenus par action d'un produit de formule générale:

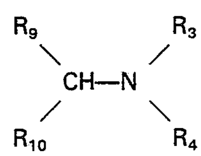

(X)

éventuellement préparé in situ, [pour lequel $R_3$ et $R_4$ sont définis comme précédemment et $R_9$ et $R_{10}$ (qui sont identiques ou différents)] soit représentent des groupements de formule générale:

$$—X_2R_{11} \quad (XIa)$$

(dans laquelle $X_2$ représente un atome d'oxygène et $R_{11}$ représente un radical alcoyle ou phényle) soit representent l'un un radical de formule générale (XIa) (dans lequel $X_2$ représente un atome d'oxygène ou de soufre et $R_{11}$ est alcoyle ou phényle), et l'autre un radical amino de formule générale:

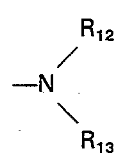

(XIb)

(dans laquelle $R_{12}$ et $R_{13}$ sont définis comme $R_3$ et $R_4$) soit encore $R_9$ et $R_{10}$ représentent chacun un radical de formule générale (XIb)] sur un dérivé d'oxacéphalosporine de formule générale:

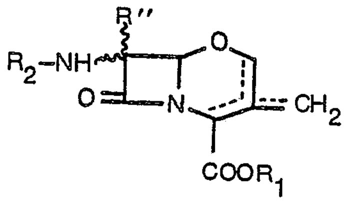

(XII)

dans laquelle, R'', $R_1$ et $R_2$ étant définis comme précédemment dans la formule générale (I), le produit se présente sous forme méthyl-3 bicyclooctène-2 ou -3 ou méthylène-3 bicyclooctane.

Lorsque l'on utilise un produit de formule générale (X) dans laquelle le radical (XIb) est différent de $—NR_3R_4$, il est préférable de choisir un tel produit de manière que l'amine $HNR_{12}R_{13}$ soit plus volatile que $HNR_3R_4$.

On opère généralement dans un solvant organique tel qu'un amide (par exemple le diméthylformamide, le diméthylacétamide ou l'hexaméthylphosphorotriamide), un nitrile (par exemple l'acétonitrile), un ester (par exemple l'acétate d'éthyle), un éther (par exemple le dioxanne), un solvant chloré (par exemple le dichloro-1,2 éthane), ou encore dans un mélange de tels solvants à une température comprise entre 20°C et la température de reflux du mélange réactionnel.

Il est entendu que lorsque $R_2$ représente un radical de formule générale (II) dans laquelle $R_5$ est un atome d'hydrogène, il est préférable que l'oxime soit protégée par un radical tel qu'indiqué précédemment.

Il est également entendu que lorsque $R_2$ contient un substituant hydroxy, il est préférable de protéger ce dernier.

L'introduction et l'élimination des radicaux protecteurs s'effectuent dans les conditions décrites dans la littérature.

Les produits de formule générale (X) peuvent être préparés selon les méthodes décrites par H. BREDERECK et coll., Chem. Ber. *101* 41 (1968), Chem. Ber. *101*, 3058 (1968), et Chem. Ber. *106*, 3725 (1973).

Les dérivés de l'oxacéphalosporine de formule générale (XII) dans laquelle $R_2$ représente un radical de formule générale (II) ou un radical α-carboxyarylacétyle dont les fonctions amine et/ou acide sont protégées peuvent être préparés à partir des produits de formule générale:

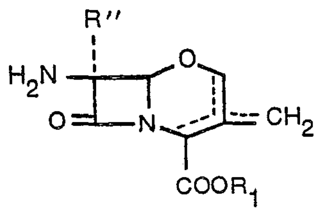

(XIII)

(dans laquelle $R_1$ est défini comme précédemment et R'' est un atome d'hydrogène ou un radical méthoxy en position 7α) par action d'un acide de formule générale:

$$R_2OH \qquad (XIV)$$

dans laquelle $R_2$ est défini comme ci-dessus, ou d'un de ses dérivés réactifs, puis éventuellement élimination du radical protecteur de l'oxime ou du radical hydroxy.

Lorsque $R_2$ représente un radical de formule générale (II), l'acide de formule générale (XIV), sous forme syn ou anti ou leurs mélanges, conduit respectivement aux produits de formule générale (XII) de forme syn ou anti ou leurs mélanges.

Il est entendu que l'oxime est préalablement protégée lorsque $R_2$ est un radical de formule générale (II) dans laquelle $R_5$ est un atome d'hydrogène.

Lorsque $R_2$ est un radical α-carboxy p.hydroxyphénylacétyle, le radical hydroxy peut être libre ou protégé.

a) Lorsque l'on utilise le produit de formule générale (XIV) sous forme acide, on effectue généralement la condensation de ce produit (dont le cas échéant l'oxime a été préalablement protégée) sur l'amino-7 oxacéphalosporine de formule générale (XIII) dans laquelle, R'' étant défini comme précédemment, $R_1$ représente un radical protecteur d'acide facilement éliminable, en opérant dans un solvant organique tel que le diméthylformamide, l'acétonitrile, le tétrahydrofuranne, le dichlorométhane ou le chloroforme, en présence d'un agent de condensation tel qu'un carbodiimide (par exemple le dicyclohexylcarbodiimide), le NN'-carbonyldiimidazole ou l'éthoxy-2 éthoxycarbonyl-1 dihydro-1,2 quinoléine, à une température comprise entre −20 et 40°C puis on élimine les groupements protecteurs présents dans la molécule. Eventuellement, on opère en présence d'une quantité catalytique de N,N-diméthylamino-4 pyridine.

b) Lorsque l'on utilise un dérivé réactif de l'acide de formule générale (XIV), il est possible de mettre en oeuvre l'anhydride, un anhydride mixte ou un ester réactif de formule générale:

$$R_2—OZ \qquad (XV)$$

dans laquelle $R_2$ est défini comme ci-dessus et Z représente un radical succinimido, benzotriazolyle-1, nitro-4 phényle, dinitro-2,4 phényle, pentachlorophényle ou phtalimido.

Il est également possible de mettre en oeuvre un dérivé réactif tel qu'un halogénure d'acide.

Lorsque l'on met en oeuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par exemple acétone), ou dans des mélanges de tels solvants en présence d'un accepteur d'acide tel qu'un époxyde (par exemple l'oxyde de propylène) ou tel qu'une base organique azotée comme la pyridine, la diméthylaminopyridine, la N-méthylmorpholine ou une trialcoylamine (par exemple triéthylamine), ou dans un milieu hydroorganique en présence d'un agent alcalin de condensation tel que le bicarbonate de sodium, et l'on opère à une temperature comprise entre −40 et +40°C, puis on remplace éventuellement les groupements protecteurs par des atomes d'hydrogène.

Lorsque l'on met en oeuvre un ester réactif de formule générale (XV), on opère généralement en présence d'une trialcoylamine (par exemple triéthylamine) dans un solvant organique tel que le diméthylformamide à une température comprise entre 0 et 40°C, puis on remplace les groupements protecteurs par des atomes d'hydrogène.

Les conditions d'élimination des groupements protecteurs sont telles que décrites ci-après pour la préparation des oxacéphalosporines de formule générale (XX).

Les oxacéphalosporines de formules générales (XII) et (XIII) peuvent être prepparées selon (ou par analogie avec) une méthode décrite dans la littérature, par exemple

— lorsque R'' représente un atome d'hydrogène: selon les méthodes décrites par C. L. BRANCH et coll., J. C. S. Perkin I, 2268 (1979), dans la demande de brevet allemand 2 806 457, dans le brevet américain 4 108 992, dans la demande de brevet allemand 2 355 209 puis éventuellement mise en place du radical $R_2$ (lorsque l'on veut obtenir un produit de formule générale (XII), par analogie avec les méthodes employées en chimie des céphalosporines et par exemple:

— lorsque $R_2$ est radical formyle: selon J. C. SHEEHAN et coll., J. Amer. Chem. Soc. *80* 1154 (1958),

— lorsque $R_2$ est acétyle, chloracétyle, trichloracétyle, phénylacétyle, phénoxyacétyle ou benzyle: selon E. H. FLYNN, Cephalosporins and Penicillins, Ac. Press (1972),

— lorsque $R_2$ est un radical t.butoxycarbonyle: selon L. MORODER et coll., Hoppe Seyler's Z. Physiol. Chem. *357* 1651 (1976),

— lorsque $R_2$ est trichloro-2,2,2 diméthyl-1,1 éthoxycarbonyle: selon J. UGI et coll., Angew. Chem. Int. Ed. Engl. *17 (5)*, 361 (1978),

— lorsque $R_2$ est trichloro-2,2,2 éthoxycarbonyle, chloro-2 diméthyl-1,1 éthoxycarbonyle, cyano-2 diméthyl-1,1 éthoxycarbonyle, triméthylsilyl-2 éthoxycarbonyle, benzyloxycarbonyle, p.méthoxybenzyloxycarbonyle, diméthoxy-3,5 benzyloxycarbonyle, p.nitrobenzyloxycarbonyle, vinyloxycarbonyle: par action d'un chloroformiate en milieu hydroorganique en présence d'un bicarbonate alcalin, ou selon le brevet belge 788 885,

— lorsque $R_2$ est diphénylméthoxycarbonyle: par action de l'azidoformiate correspondant en milieu hydroorganique, en présence d'un bicarbonate alcalin,

— lorsque $R_2$ est (biphénylyl-4)-2 isopropyloxycarbonyle: par analogie avec la méthode décrite dans Helv. Chim. Acta, *51*, 924 (1968),

— lorsque $R_2$ est quinolyl-8 oxycarbonyle ou allyloxycarbonyle: par action du carbonate correspondant en milieu hydroorganique basique,

— lorsque $R_2$ est o-nitrophénylthio ou p-nitrophénylthio: par analogie avec la méthode décrite par T. KOBAYASHI et coll., Chem. Pharma. Bull. *27(11)*, 2718 (1979)

— lorsque $R_2NH$ est remplacé par diméthylaminométhylèneamino: par analogie avec la méthode décrite par J. F. FITT, J. Org. Chem. *42(15)*, 2639 (1977),

— lorsque $R_2NH$ est remplacé par nitro-4 benzylidèneamino ou diméthoxy-3,4 benzylidèneamino: selon la méthode décrite par R. A. FIRESTONE, Tetrahedron Lett., *375* (1972),

— Lorsque $R_2NH$ est remplacé par di t.butyl-3,5 hydroxy-4 benzylidèneamino: selon la méthode décrite par H. YANAGISAWA et coll., Tet. Lett., 2705 (1975).

— lorsque R'' représente un radical méthoxy: par analogie avec les méthodes décrites précédemment pour la préparation des produits de formule générale (XII) ou (XIII) ou selon la demande de brevet allemand 2 806 457, puis lorsque l'on veut obtenir un produit de formule générale (XII) mise en place du radical $R_2$ par analogie avec les méthodes citées ci-dessus.

Les nouveaux produits de formule générale (I) sont utiles comme intermédiaires pour la préparation de thiovinyl-3 oxacéphalosporines de formule générale:

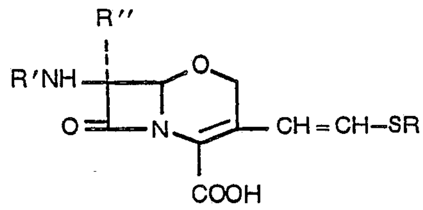

(XVI)

dans laquelle le symbole R est choisi parmi les significations:

1) pyridyle-2, -3 ou -4 éventuellement N-oxydés

2) pyrimidinyle-2

3) méthyl-6 oxyde-1 pyridazinyle-3

4) dioxo-5,6 tétrahydro-1,4,5,6 triazine-1,2,4 yle-3 substitué en position -4 par

a) un radical alcoyle contenant 1 ou 2 atomes de carbone éventuellement substitué par un radical alcoyloxy, alcoylthio, formyle,

b) un radical allyle, dihydroxy-2,3 propyle, dihydroxy-1,3 propyle-2 ou formyl-2 hydroxy-2 éthyle,

c) un radical alcoyle contenant 2 ou 3 atomes de carbone substitué par hydroxy, carbamoyloxy, acyloxy ou acylamino (dont les portions acyles sont non substituées ou substituées par amino), alcoylsulfonylamino, uréido, alcoyluréido ou dialcoyluréido,

5) dioxo-5,6 tétrahydro-1,4,5,6 triazine-1,2,4 yle-3 substitué en position -1 ou dioxo-5,6 tétrahydro-1,2,5,6 triazine-1,2,4 yle-3 substitué en position -2 par un radical alcoyle contenant 1 ou 2 atomes de carbone éventuellement substitué par un radical formyle

6) alcoyl-2 dihydro-2,5 oxo-5 triazine-1,2,4 yle-3 éventuellement substitué en position -6 par un radical alcoyle, alcoyloxy dont les portions et radicaux alcoyles contiennent 1 ou 2 atomes de carbone

7) amino-1 dihydro-1,2 oxo-2 pyrimidinyle-4

8) thioadiazol-1,3,4 yle-5 substitué par alcoyle, dialcoylaminoalcoyle ou acylaminoalcoyle,

9) tétrazolyle-5 substitué en position -1 par

a) un radical alcoyle contenant 1 ou 2 atomes de carbone éventuellement substitué par un radical formyle,

b) un radical alcoyle contenant 2 ou 3 atomes de carbone substitué par hydroxy, acylamino ou dialcoylamino, ou

c) par un radical dihydroxy-2,3 propyle ou dihydroxy-1,3 propyle-2

10) a) alcoyl-1 triazol-1,2,4 yle-5 éventuellement substitué en position -3 par un radical alcoyloxycarbonyle dont les radicaux alcoyle et alcoyloxy contiennent 1 ou 2 atomes de carbone,

b) alcoyl-1 triazol-1,3,4 yle-5,

le symbole R′ représente un radical de formule générale:

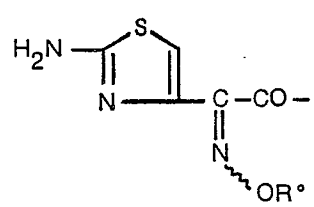

(IIc)

[dans laquelle R° est un atome d'hydrogène, un radical alcoyle, vinyle, un radical de formule générale (III)] ou un radical α-carboxyarylacétyle (dans lequel aryle représente phényle, p.hydroxyphényle ou thiényle-2 ou -3) et le symbole R″ représente un atome d'hydrogène ou un radical méthoxy en position 7α.

Il est entendu que dans les produits de formule générale (XVI), le substituant en position -3 du bicyclooctène présente la stéréoisomérie E ou Z et que le radical de formule générale (IIc) peut se présenter sous les formes syn ou anti.

Les produits de formule générale (XVI) peuvent être obtenus à partir des produits de formule générale (I) en opérant de la manière suivante:

Les produits de formule générale:

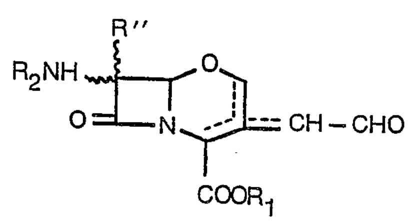

(XVII)

dans laquelle $R_1$, $R_2$ et R'' sont définis comme précédemment (étant entendu que, lorsque $R_2$ contient un radical carboxy, ce dernier peut être libre ou protégé) et qui se présentent sous forme bicyclooctène-2 ou -3 oxoéthylidène-3 bicyclooctane sont préparés par hydrolyse de l'énamine (ou du mélange d'énamines isomères) de formule générale (I).

On o-ère généralement dans un acide organique (par exemple acide formique, acide acétique) ou minéral (par exemple acide chlorhydrique, acide sulfurique) en présence ou non d'un solvant, en milieu aqueux ou organique, à une température comprise entre −20°C et la température de reflux du mélange réactionnel. Lorsque l'on opère en milieu organique, l'hydrolyse est réalisée par addition d'eau au mélange réactionnel, puis on traite éventuellement par une base minérale (par exemple bicarbonate alcalin) ou organique (par exemple amine tertiaire ou pyridine).

Lorsque l'on opère en présence d'un solvant, il n'est pas nécessaire que le solvant soit miscible à la phase aqueuse acide. Le contact est alors réalisé par agitation vive.

Parmi les solvants utilisables, peuvent être cités les solvants chlorés, l'acétate d'éthyle, le tétrahydrofuranne, l'acétonitrile, le diméthylformamide, les alcools.

Lorsque l'on veut obtenir un aldéhyde de formule générale (XVII) dans laquelle $R_2$ contient une fonction acide libre, il est nécessaire d'opérer à partir d'une énamine dans laquelle $R_1$ et le groupement protecteur de la fonction acide de $R_2$ sont différents et éliminables sélectivement.

L'élimination du radical protecteur s'effectue dans des conditions qui seront décrites ci-après.

Il n'est pas absolument nécessaire d'avoir purifié l'énamine de formule générale (I) pour la mettre en oeuvre dans cette réaction.

Les produits de formule générale:

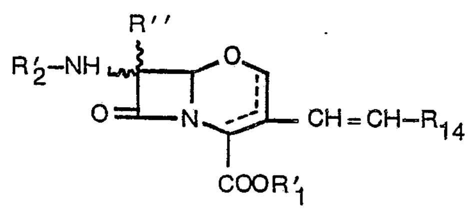

(XVIII)

{qui se présentent sous forme bicyclooctène-2 ou -3, dans laquelle

α) $R'_1$ est uin atome d'hydrogène ou un radical protecteur facilement éliminable, $R'_2$ est un radical de formule générale (II) dans laquelle $R_5$ est un atome d'hydrogène, un radical alcoyle, vinyle ou carboxyalcoyle défini par la formule générale (III) (libre ou protégé), et $R_6$ est un atome d'hydrogène ou un radical protecteur, ou $R'_2$ est un radical α-carboxyarylacétyle tel que défini précédemment en a) et dans lequel les radicaux carboxy et hydroxy sont libres ou protégés, et R'' est un atome d'hydrogène ou un radical méthoxy en 7α, ou bien

β) $R'_1$ est un radical protecteur facilement éliminable et $R'_2$ et R'' sont définis comme précédemment en b) pour la formule générale (I) et $R_{14}$ représente un radical de formule générale

$$-O-SO_2-R_{15} \quad \text{ou} \quad -OCO-R'_{15}$$

(XI Xa) (XIXb)

[dans lesquelles $R_{15}$ est un radical alcoyle, trifluorométhyle, trichlorométhyle ou un radical phényle éventuellement substitué par un atome d'halogène ou par un radical alcoyle ou nitro, et $R'_{15}$ est défini comme $R_{15}$ ou représente un radical acylméthyle, acyl-2 éthyle, acyl-2 propyle, alcoyloxycarbonylméthyle, alcoyloxycarbonyl-2 éthyle ou alcoyloxycarbonyl-2 propyle] ou un atome d'halogène choisi parmi chlore, brome et iode, et dont le substituant sur l'atome de carbone en position -3 du bicyclooctène présente la stéréoisomérie E ou Z} sont préparés par action d'un dérivé des acides $R_{15}SO_3H$ ou $R'_{15}COOH$ du type

$$\left.\begin{array}{ll} (R_{15}SO_2)_2O & (a) \\ R_{15}SO_2Hal & (b) \\ (R'_{15}CO)_2O & (c) \\ R'_{15}COHal & (d) \end{array}\right\} \quad (XX)$$

($R_{15}$ et $R'_{15}$ étant définis comme précédemment et Hal étant un atome d'halogène) ou d'un agent

d'halogénation sur un produit de formule générale (XVII) (ou sur un mélange de ses isomères), suivie éventuellement de l'élimination des radicaux protecteurs.

Lorsque l'on désire mettre en oeuvre un produit de formule générale (XVII) dans laquelle $R_2$ est un radical de formule générale (II) dont le reste $R_5$ est un atome d'hydrogène, il est nécessaire de protéger préalablement l'oxime.

Lorsque l'on désire mettre en oeuvre un aldéhyde de formule générale (XVII) dans laquelle $R_2$ contient un groupement carboxy, ce radical peut être libre ou protégé si l'on fait agir un dérivé activé des acides $R_{15}SO_3H$ ou $R'_{15}COOH$; par contre il est nécessaire de le protéger préalablement si l'on fait agir un agent d'halogénation.

Lorsque l'on désire mettre en oeuvre un aldéhyde dans lequel le radical $R_2$ est α-carboxy (p.hydroxyphényl)acétyle, il est nécessaire de protéger le radical hydroxy.

La protection des radicaux s'effectue dans les conditions décrites précédemment.

On opère généralement en présence d'une base tertiaire représentée par la formule générale:

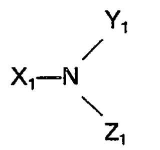

(XXI)

dans laquelle $X_1$, $Y_1$ et $Z_1$ représentent des radicaux alcoyle ou phényle, ou éventuellement 2 d'entre eux forment un cycle avec l'atome d'azote auquel ils sont rattachés: on utilise par exemple la triéthylamine ou la NN-diméthylaniline dans un solvant organique chloré (par exemple dichlorométhane), dans un ester (acétate d'éthyle), dans un éther (par exemple dioxanne, tétrahydrofuranne), dans un amide (par exemple diméthylacétamide, diméthylformamide), dans l'acétonitrile ou la N-méthylpyrrolidone ou dans un mélange de tels solfants ou directement dans un solvant basique comme la pyridine, ou bien lorsque $R_{14}$ est autre qu'un atome d'halogène, ou peut opérer en milieu hydroorganique en présence d'un agent alcalin de condensation (par exemple bicarbonate alcalin, soude ou potasse), à une température comprise entre −78°C et la température de reflux du mélange réactionnel.

Eventuellement on opère sous azote.

Il n'est pas absolument nécessaire d'avoir purifié l'intermédiaire de formule générale (XVII) pour mettre en oeuvre cette réaction.

Lorsque l'on veut préparer un produit de formule générale (XVIII) dans laquelle $R_{14}$ est un atome d'halogène, les agents d'halogénation peuvent être choisis parmi les dérivés halogénés du phosphore, notamment:

— les composés d'addition d'halogène et de triarylphosphite, ou bien

— le trichlorure de phosphore, l'oxychlorure de phosphore, le pentachlorure de phosphore, le dichlorotriphénylphosphorane ou le catéchyltrichlorophosphorane lorsque $R_{14}$ est un atome de chlore ou

— le tribromure de phosphore, l'oxybromure de phosphore, le pentabromure de phosphore ou le catéchyltribromophosphorane lorsque $R_{14}$ est un atome de brome.

Le catéchyltrichloro (ou tribromo) phosphorane, qui peut être préparé in situ, peut être obtenu selon la méthode décrite par H. GROSS et U. KARSCH, J. Prakt. Chem, *29*, 315 (1965).

Les composés d'addition d'halogène et de triarylphosphite, qui peuvent être préparés in situ, sont décrits par H. N. RYDON et B. L. TONGE, J. Chem. Soc., 3043 (1956), par J. MICHALSKI et coll., J. Org. Chem., *45*, 3122 (1980) ou dans le brevet belge 881 424 et peuvent être préparés selon les méthodes mentionnées dans ces documents.

La préparation des dérivés halogénés de formule générale (XVIII) s'effectue en milieu anhydre.

Lorsque l'on veut préparer un produit de formule générale (XVIII), dans laquelle $R_{14}$ est un atome de chlore ou de brome, selon les conditions opératoires on peut isoler l'intermédiaire dihalogéné de formule générale:

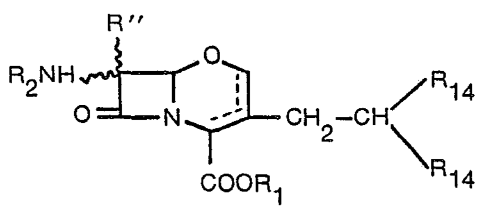

(XXII)

[dans laquelle R'', $R_1$, $R_2$ et $R_{14}$ étant définis comme ci-dessus, le produit présente la même isomérie que le produit de formule générale (XVIII)] qui est ensuite déhydrohalogéné.

Lorsque l'on veut isoler l'intermédiaire dihalogéné, on opère par action d'un agent d'halogénation, dans un solvant organique tel qu'un solvant chloré (par exemple dichlorométhane, chloroforme, tétrachlorure de carbone, dichloro-1,2 éthane), un éther (par exemple éther éthylique, oxyde de propylène, tétrahydrofuranne, dioxanne), un amide (par exemple diméthylacétamide, diméthylpropionamide,

diméthylformamide, N-acétylmorpholine, N-acétylpipéridine, N-méthylpyrrolidone) ou un mélange de tels solvants, à une température un peu moins élevée que pour préparer le dérivé halogénovinyl correspondant, c'est-à-dire comprise entre −78 et 30°C.

Il est également possible d'opérer en présence d'une base telle que la pyridine dans un solvant cité ci-dessus, à une température comprise entre −78 et 0°C.

La déhydrohalogénation s'effectue en présence d'une base tertiaire telle que définie précédemment, d'une amine aromatique (par exemple pyridine, picoline, quinoléine) ou d'une base minérale (telle que la soude, la potasse, un carbonate ou un bicarbonate alcalin o alcalino-terreux), en milieu organique ou hydroorganique dans les solvants cités précédemment, à une température comprise entre −20°C et la température de reflux du mélange réactionnel.

Il n'est pas absolument nécessaire d'avoir purifié l'intermédiaire dihalogéné pour procéder à sa déhydrohalogénation.

Le cas échéant, l'élimination des radicaux protecteurs peut s'effectuer simultanément ou successivement.

A titre d'exemple,

1) L'élimination des groupements protecteurs d'amines s'effectue

— lorsqu'il s'agit d'un radical t.butoxycarbonyle, trityle, p-méthoxybenzyloxycarbonyle ou formyle: par traitement en milieu acide. On utilise par exemple l'acide trifluoracétique en opérant à une température comprise entre 0 et 20°C, ou bien on utilise l'acide formique, phosphorique ou polyphosphorique purs ou en présence d'eau à une température comprise entre 20 et 60°C, ou encore l'acide paratoluènesulfonique ou méthanesulfonique dans l'acétone ou l'acétonitrile, à une température comprise entre 20°C et la température de reflux du mélange réactionnel. Dans ces conditions lorsque $R'_2$ est un radical de formule générale (II) le produit de formule générale (XVIII) peut être obtenu sous forme de trifluoracétate, de solvate avec l'acide formique, de phosphate, de méthanesulfonate ou de paratoluènesulfonate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique;

— lorsqu'il s'agit d'un radical trichloro-2,2,2 éthoxycarbonyle ou p.nitrobenzyloxycarbonyle: par réduction (notamment traitement par le zinc dans l'acide acétique);

— lorsqu'il s'agit d'un radical chloracétyle ou trichloracétyle: par application de la méthode décrite dans le brevet français publié sous le n° 2 243 199;

— lorsqu'il s'agit d'un radical benzyle, dibenzyle ou benzyloxycarbonyle: par hydrogénation catalytique;

— lorsqu'il s'agit d'un radical trifluoroacétyle: par traitement en milieu basique.

2) L'élimination des groupements protecteurs du radical carboxy s'effectue:

— lorsqu'il s'agit d'un groupement t.butyle, p.méthoxybenzyle ou benzhydryle: par traitement en milieu acide, dans les conditions décrites ci-dessus pour l'élimination du radical trityle protecteur d'amino. Dans le cas du radical benzhydryle, on peut opérer en présence d'anisole;

— lorsqu'il s'agit d'un groupement méthoxyméthyle: par traitement en milieu acide dilué;

— lorsqu'il s'atig d'un groupement nitrobenzyle: par réduction (notamment traitement par le zinc dans l'acide acétique ou hydrogénolyse).

3) L'élimination des groupements protecteurs de l'oxime et/ou des radicaux hydroxy s'effectue

— lorsqu'il s'agit de groupement trityle ou tétrahydropyrannyle: par acidolyse, par exemple par l'acide trifluoracétique, l'acide formique aqueux ou non, ou l'acide paratoluènesulfonique. Lorsqu'on utilise l'acide formique, aqueux ou non, la libération des radicaux hydroxy protégés à l'état d'acétal cyclique peut conduire au moins partiellement aux mono ou diesters correspondants, qui peuvent être séparés le cas échéant par chromatographie;

— lorsqu'il s'agit du groupement méthoxy-2 propyle-2: selon la méthode décrite dans le brevet belge 875 379.

— lorsqu'il s'agit des groupements alcoyloxycarbonyle ou aryloxycarbonyle: selon les méthodes décrites dans le brevet belge 871 213.

Les produits de formule générale (XVIII) pour lesquels $R'_1$, $R'_2$ et R″ sont définis comme précédemment en a), qui seront représentés ci-après par la formule générals:

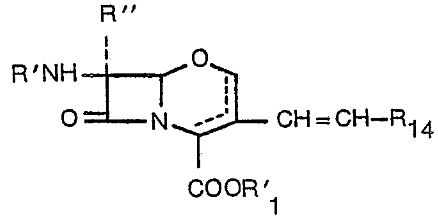

(XVIIIa)

sont aussi obtenus en opérant de la manière suivante:

On élimine le radical protecteur $R'_2$ d'un produit de formule générale (XVIII) dans laquelle $R'_1$ et $R'_2$ sont définis comme précédemment en β) et R'' est un atome d'hydrogène ou un radical méthoxy en 7α (ou éventuellement on élimine simultanément ou successivement les radicaux protecteurs $R'_2$ et $R'_1$), pour préparer une amino-7 oxacéphalosporine de formule générale:

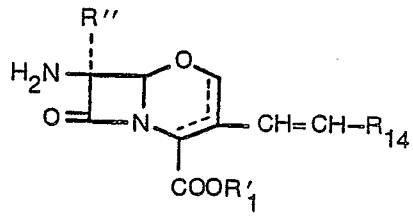

(XXIII)

dans laquelle $R'_1$ est atome d'hydrogène ou un radical protecteur d'acide facilement éliminable et R'' est un atome d'hydrogène ou un radical méthoxy en 7α et qui présente la même stéréoisomérie que les produits de formule générale (XVIII).

Puis on fait agir un acide de formule générale

$$R'—OH \qquad (XXIV)$$

dans laquelle R' est défini comme $R'_2$ précédemment en α) pour les produits de formule générale (XVIII) et dont le cas échéant la fonction amine a été préalablement protégée, ou un dérivé réactif de cet acide, sur cette amino-7 oxacéphalosporine de formule générale (XXIII), et on élimine éventuellement les radicaux protecteurs.

L'élimination du radical protecteur $R'_2$ du produit de formule générale (XVIII) s'effectue par toute méthode connue pour libérer une fonction amine sans toucher au reste de la molécule.

A titre d'exemple, on peut citer les méthodes suivantes:

— lorsque $R'_2$ représente trityle, benzhydryle, trichloracétyle, chloracétyle, t.butoxycarbonyle, trichloréthoxycarbonyle, benzyloxycarbonyle, p.méthoxybenzyloxycarbonyle et p.nitrobenzyloxycarbonyle: selon les méthodes citées ci-dessus pour la libération du radical amino du produit de formule générale (I):

— lorsque $R'_2$ représente formyle, chloro-2 diméthyl-1,1 éthoxycarbonyle, cyano-2 diméthyl-1,1 éthoxycarbonyle, diméthoxy-3,5 benzyloxycarbonyle, diphénylméthoxycarbonyle, (biphénylyl-4)-2 isopropyloxycarbonyle, vinyloxycarbonyle, allyloxycarbonyle, quinolyl-8 oxycarbonyle, o.nitrophénylthio, p.nitrophénylthio, et lorsque $R'_2NH$— est remplacé par diméthylaminométhylèneamino, diméthoxy-3,4 benzylidèneamino ou nitro-4 benzylidèneamino: par hydrolyse en milieu acide;

— lorsque R82 représente di t-butyl-3,5 hydroxy-4 benzylidèneamino: par traitement par le réactif T de GIRARD par analogie avec la méthode décrite dans le brevet belge 863 998;

— lorsque $R'_2$ représente trichloro-2,2,2 éthyle ou trichloro-2,2,2 diméthyl-1,1 éthoxycarbonyle: par traitement par le zinc dans l'acide acétique;

— lorsque $R'_2$ représente acétyle, benzoyle, phénylacétyle ou phénoxyacétyle: selon la méthode decrite dans le brevet belge BE 758 800 ou selon la méthode décrite par YOSHIOKA, Tet. Letters 351 (1980);

— lorsque $R'_2$ représente triméthylsilyléthoxycarbonyle: selon la méthode décrite par H. GERLACH, Helv.Chim. Acta *60* (8), 3039 (1977);

— lorsque R82 représente p.nitrobenzyloxycarbonyle ou benzyle: par hydrogénolyse en présence de palladium.

Lorsque R'' est un groupe méthoxy on pourra, de préférence aux méthodes précédentes, utiliser les méthodes suivantes:

1) Dans le cas où $R'_2$ représente benzhydryle ou trityle: hydrogénolyse en présence de palladium.

2) Dans le cas où $R'_2$ représente des radicaux de formules générales (VII) ou (VIII): par les méthodes décrites par E. M. GORDON, J. Am. Chem. Soc. *102*(5), 1690 (1980); T. KOBAYASHI, Bull. Chem. Soc. Japan *52*(11), 3366 (1979) et T. KOBAYASHI, Chem. Pharm. Bull. *27*, 2718 (1979).

3) Dans le cas où représente un radical de formule générale (IX): par acidolyse ou par traitement par le réactif T de Girard, par analogie avec la méthode décrite dans le brevet belge 863 998.

La réaction de l'acide de formule générale (XXIV), ou de son dérivé réactif, avec l'amino-7 oxacéphalosporine de formule générale (XXIII), s'effectue dans les conditions décrites précédemment pour l'action d'un acide de formule générale (XIV) ou de son dérivé réactif sur une amino-7 céphalosporine de formule générale (XIII).

Lorsque R' représente un radical de formule générale (II), il est entendu que l'oxime est protégée lorsque $R_5$ représente un atome d'hydrogène et que, lorsque $R_5$ contient un radical carboxy, celui-ci est également protégé.

Lorsque R' représente un radical α-carboxyarylacétyle la protection du groupement carboxy n'est pas obligatoire; il peut donc être libre ou protégé.

Il en est de même pour le radical hydroxy lorsque le groupement aryle représente p.hydroxyphényle.

Lorsque l'on met en oeuvre un dérivé réactif de l'acide de formule générale (XXIV) tel qu'un halogénure d'acide, on peut faire réagir le chlorhydrate du chlorure d'acide, on peut faire réagir le chlorhydrate du chlorure d'acide lorsque R' est un radical de formule générale (II).

Le cas échéant l'élimination des radicaux protecteurs peut être effectuée dans les conditions décrites précédemment.

Les thiovinyl-3 oxacéphalosporines de formule générale (XVI) peuvent alors être obtenues en opérant de la manière suivante:

A) On fait agir un thiol de formule générale:

$$R{-}SH \qquad (XXV)$$

{dans laquelle R, qui est défini comme précédemment pour la formule générale (XVI), est protégé à l'état d'acétal lorsque l'on veut obtenir une oxacéphalosporine dans laquelle R contient un radical formyle} (ou un de ses sels alcalins ou alcalino-terreux) sur un dérivé d'oxacéphalosporine (ou un mélange de ses isomères) de formule générale (XXIIIa) dans laquelle $R'_1$, R' et R'' sont définis comme précédemment, puis on élimine le cas échéant les radicaux protecteurs.

Lorsque l'on veut obtenir un produit de formule générale (XVI) dans laquelle R contient un radical formylalcoyle, on met en oeuvre un thiol dans lequel R est protégé à l'état d'acétal, sous forme d'un radical de formule générale:

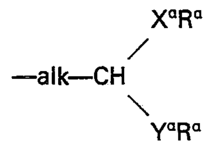

(XXVIa)

ou

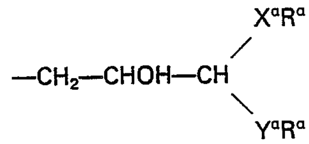

(XXVIb)

dans lesquelles alk est un radical alcoylène contenant 1 à 4 atomes de carbone, $X^\alpha$ et $Y^\alpha$ sont identiques et représentent des atomes d'oxygène ou de soufre, et $R^\alpha$ représente un radical alcoyle, ou bien $X^\alpha$ et $Y^\alpha$ sont identiques ou différents et représentent des atomes d'oxygène ou de soufre, et les radicaux $R^\alpha$ forment ensemble un radical alcoylène contenant 2 ou 3 atomes de carbone.

Lorsque le radical R du produit de formule générale (XXV) est susceptible d'interférer avec la réaction, il est préférable de protéger ce groupement dans les conditions décrites précédemment (notamment lorsque R contient un radical amino, alcoylamino, hydroxy ou carboxy).

Les radicaux dihydroxy-2,3 propyle ou dihydroxy-1,3 propyle-2 peuvent être (ou sont) protégés sous forme d'un radical diméthyl-2,2 dioxolannyl-4 méthyle ou diméthyl-2,2 dioxannyle-5.

Lorsque le radical R' représente un groupe de formule générale (II), le radical amino est libre ou protégé, lorsque $R_5$ représente un atome d'hydrogène, l'oxime est de préference protégée et lorsque $R_5$ contient un groupement carboxy celui-ci est libre ou protégé.

Lorsque le radical R' représente un groupe α-carboxyarylacétyle, il est préférable de protéger le radical hydroxy lorsque le substituant aryle est p.hydroxyphényle; le groupement carboxy peut être libre ou protégé.

La protection et l'élimination de tous ces radicaux s'effectuent par exemple par l'un des groupements définis précédemment et dans les conditions décrites précédemment.

L'élimination du groupement protecteur de R est effectuée avant, simultanément ou après l'élimination des autres radicaux protecteurs.

L'élimination des groupements protecteurs contenus dans les radicaux de formule générale (XXVIa ou b) (lorsque l'on veut obtenir un produit de formule générale (XVI) dans laquelle R contient un radical formylalcoyle) s'effectue par exemple:

— en présence d'un acide sulfonique (par exemple acide méthanesulfonique ou acide p.toluènesulfonique) dans un solvant organique (par exemple acétonitrile ou acétone), éventuellement en présence d'eau et éventuellement en présence d'un réactif acétalisable tel que l'acétone, l'acide glyoxylique, le benzaldéhyde ou l'acide pyruvique, à une température comprise entre 20°C et la température de reflux du mélange réactionnel;

— ou bien, lorsque le radical R doit être un radical dioxo-5,6 tétrahydro-1,4,5,6 triazine-1,2,4 yle-3, par action d'acide formique aqueux (contenant de préférence moins de 10% d'eau), soit en présence ou non de silice, soit par transacétalisation en présence d'un réactif acétalisable tel que défini ci-dessus.

La transformation des groupements protégés diméthyl-2,2 dioxolannyl-4 méthyle ou diméthyl-2,2 dioxannyle-5 respectivement en radicaux dihydroxy-2,3 propyle ou dihydroxy-1,3 propyle-2 s'effectue, à titre d'exemple, par acidolyse, notamment par l'acide trifluoracétique, l'acide formique aqueux ou non ou l'acide paratoluènesulfonique.

Lorsqu'on utilise l'acide formique, aqueux ou non, la libération des radicaux hydroxy protégés à l'état d'acétal cyclique peut conduire au moins partiellement aux mono ou diesters correspondants, qui peuvent être séparés le cas échéant par chromatographie; on opère généralement en présence d'une base

organique telle qu'une pyridine ou une base organique tertiaire de formule générale (XXI). On utilise par exemple la diisopropyléthylamine ou la diéthylphénylamine.

Lorsque l'on fait agir un sel alcalin ou alcalinoterreux du thiol de formule générale (XXVI), il n'est pas nécessaire d'opérer en présence d'une base organique telle que définie ci-dessus.

La réaction s'effectue avantageusement dans un solvant organique tel que le diméthylformamide, le tétrahydrofuranne ou l'acétonitrile ou un mélange de tels solvants.

Il est également possible d'opérer en présence de bicarbonate alcalin dans un solvant tel que cité ci-dessus, éventuellement en présence d'eau.

On opère à une température comprise entre −20°C et la température de reflux du mélange réactionnel, la température choisie étant variable selon le thiol employé. De même, selon le thiol employé, la durée de réaction peut varier de 5 minutes à 48 heures.

Eventuellement on opère sous azote.

De préférence, lorsque l'on veut utiliser un bicyclooctène-3 de formule générale (XVIIIa), on met en oeuvre un tel produit pour lequel $R'_1$ est autre que l'hydrogène.

B) On prépare une amino-7 oxacéphalosporine de formule générale:

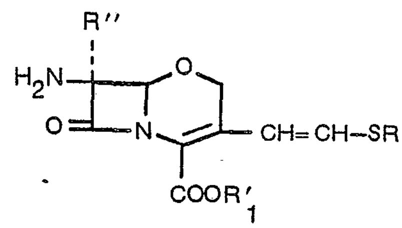

(XXVII)

{dans laquelle R est défini comme précédemment, R'' est un atome d'hydrogène ou un radical méthoxy en 7α et $R'_1$ est un atome d'hydrogène ou un radical protecteur d'acide facilement éliminable, et dont le substituant en position -3 présente la stéréoisomérie E ou Z}, puis on acyle ce produit pour obtenir un produit de formule générale (XVI). On opère successivement selon l'un des schémas suivants:

$B_1$) on prépare tout d'abord un dérivé d'oxacéphalosporine de formule générale:

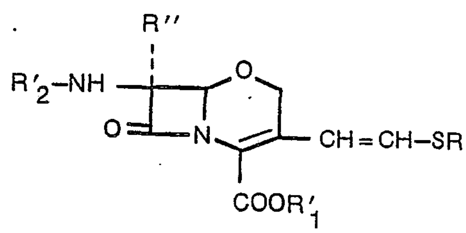

(XXVIII)

dans laquelle R est défini comme précédemment, $R'_1$ et $R'_2$ sont définis comme en β) pour les produits de formule générale (XVIII) et R'' est un atome d'hydrogène ou un radical méthoxy en 7α, en faisant agir un thiol de formule générale (XXV) (ou un de ses sels alcalins ou alcalino-terreux) sur un dérivé d'oxacéphalosporine de formule générale (XVIII) dans laquelle $R'_1$, $R'_2$ et R'' sont définis comme ci-dessus (ou le cas échéant sur un mélange des isomères de ce produit).

La réaction s'effectue généralement dans les conditions décrites précédemment pour l'obtention d'une thiovinyl-3 oxacéphalosporine de formule générale (XVI) à partir d'une oxacéphalosporine de formule générale (XVIII) et d'un thiol de formule générale (XXV). Puison élimine le radical protecteur $R'_2$ du produit de formule générale (XXVIII) (ou éventuellement on élimine successivement ou simultanément le radical $R'_2$ et les autres radicaux protecteurs, pour préparer l'amino-7 oxacéphalosporine de formule générale (XXVII).

On opère dans les conditions décrites précédemment pour la préparation des produits de formule générale (XXIII) à partir des produits de formule générale (XVIII); ou bien

$B_2$) on prépare un dérivé d'oxacéphalosporine de formule générale:

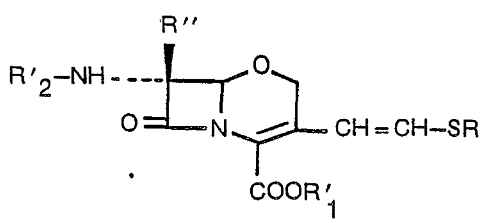

(XXVIIIa)

dans laquelle R, $R'_1$ et $R'_2$ sont définis comme dans la formule générale (XXVIII), et R″ représente un atome d'hydrogène en 7β, en faisant agir un thiol de formule générale (XXV) (ou un de ses sels alcalins ou alcalinoterreux) sur un produit de formule générale (XVIII) dans laquelle $R'_1$, $R'_2$ et R″ sont définis comme ci-dessus (ou le cas échéant sur un mélange de ses isomères).

La réaction s'effectue comme décrit précédemment pour la préparation des produits de formule générale (XXVIII).

Puis on élimine le radical protecteur $R'_2$ du produit de formule générale (XXVIIIa) (ou éventuellement le radical $R'_2$ et le radical protecteur de R) en opérant dans les conditions décrites précédemment pour la préparation du produit de formule générale (XXVII). On obtient ainsi le produit de formule générale:

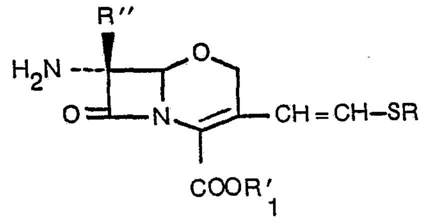

(XXVIIa)

{dans laquelle R et $R'_1$ sont définis comme pour le produit de formule générale (XXVII) et R″ est un atome d'hydrogène en 7β et dont le substituant en position -3 présente la stéréoisomérie E ou Z} qui est alors converti en le dérivé méthoxylé correspondant de formule générale (XXVII).

La réaction s'effectue dans les conditions décrites dans les brevets belges 871 213 et 863 998, ou selon la méthode décrite par T. KOBAYASHI et coll., Chem. Pharm. Bull. *27(11)*, 2718 (1979), oui la méthode décrite par H. YANAGISAWA et coll., Tet. Lett., *31*, 2705 (1975), étant entendu que les substituants du radical R susceptibles d'interférer avec la réaction sont préalablement protégés.

$B_3$) Lorsque l'on veut obtenir un produit de formule générale (XXVII) dans laquelle R″ est méthoxy on prépare un dérivé d'oxacéphalosporine de formule générale (XXVIIIa) dans laquelle $R'_2$ est un radical facilement éliminable de formule générale (IV), (VII), (VIII) ou (IX), en opérant comme décrit ci-dessus en $B_2$), puis on prépare un produit de formule générale (XXVIII) dans laquelle R″ est un radical méthoxy et $R'_2$ est défini comme ci-dessus, par méthoxylation du dérivé de formule générale (XXVIIIa) obtenu.

La méthoxylation s'effectue dans les conditions décrites dans les brevets belges 871 213 et 863 998 ou selon les méthodes décrites par T. KOBAYASHI et coll., Chem. Pharm. Bull., *27(11)*, 2718 (1979), ou par H. YANAGISAWA et coll., Tet. Lett., *31*, 2705 (1975).

On prépare alors l'amino-7 oxacéphalosporine de formule générale (XXVII) comme décrit précédemment en $B_1$). Ou bien,

$B_4$) Lorsque l'on veut obtenir un produit de formule générale (XXVII) dans laquelle R″ est méthoxy, on prépare une amino-7 oxacéphalosporine de formule générale (XXVII) dans laquelle R″ est un atome d'hydrogène, en opérant dans les conditions décrites précédemment en $B_1$, puis on transforme le produit obtenu en le produit méthoxylé correspondant en opérant dans les conditions décrites ci-dessus.

On effectue alors l'acylation du produit de formule générale (XXVII) par action d'un acide représenté par la formule générale (XXIV) dans laquelle R′ est défini comme précédemment [étant entendu que lorsque R′ est un radical de formule générale (II), la fonction amine de ce radical est protégée] ou d'un dérivé réactif de cet acide, sur une amino-7 oxacéphalosporine de formule générale (XXVII), suivie de l'élimination des radicaux protecteurs.

La réaction s'effectue dans les conditions utilisées pour la préparation des produits de formule générale (XVIIIa), les conditions de blocage étant les mêmes.

De plus lorsque dans la formule générale (XXVII) R contient un substituant amino ou alcoylamino, ce groupement est protégé, et lorsqu'il contient un substituant hydroxy, ce dernier est libre ou de préférence protégé.

Il est entendu que les groupements amino, alcoylamino, carboxy et hydroxy qui existent dans certains radicaux sont (ou peuvent être) protégés par tous groupements protecteurs habituellement utilisés pour la protection des amines, des acides carboxyliques, des alcools ou des oximes et dont la mise en oeuvre n'altère pas le reste de la molécule.

L'élimination des radicaux protecteurs s'effectue dans les conditions décrites précédemment.

Les produits de formule générale (XIV) ou (XXIV) peuvent être préparés selon la méthode décrite dans le brevet belge 850 662, ou par application de la méthode décrite dans le brevet belge 877 884, lorsque R′ est un radical de formule générale (II) dans laquelle $R_5$ est hydrogène ou alcoyle.

Les produits de formule générale (XIV) ou (XXIV) peuvent être préparés selon la méthode décrite dans le brevet belge 869 079 lorsque R′ est un radical de formule générale (II) dans laquelle $R_5$ est vinyle.

Les produits de formule générale (XIV) ou (XXIV) peuvent être préparés selon les méthodes décrites dans les brevets belges 864 810, 865 298, 876 541 et 876 542 lorsque R′ est un radical de formule générale (II) dans laquelle $R_5$ est un substituant de formule générale (III).

Les produits de formule générale (XIV) ou (XXIV), lorsque R′ est un radical α-carboxyarylacétyle, peuvent être préparés selon les méthodes suivantes:

— lorsque le groupement aryle représente un groupement p. hydroxyphényle: selon la méthode décrite dans la demande de brevet japonais 79 106 447 ou dans le brevet belge 852 912.

— lorsque le groupement aryle représente un groupement thiényle-2: selon D. IVANOV et N. MAREKOV, Compt, Rend. Acad. Bulgare Sci., *8(11)*, 29 (1955).

— lorsque le groupement aryle représente un groupement thiényle-3: selon le brevet britannique 1 125 557.

Les thiols de formule générale (XXV), qui peuvent être mis en oeuvre sous leur forme tautomère, peuvent être préparés par application de l'une des méthodes suivantes selon la signification du radical R:

— lorsque R est un radical pyridyle-3: selon la méthode décrite par H. M. WUEST et E. H. SAKAL, J. Am. Chem. Soc., *73*, 1210 (1951),

— lorsque R est un radical oxyde-1 pyridyle-3: selon la méthode décrite par B. BLANK et coll., J. Med. Chem. *17*, 1065 (1974),

— lorsque R est un radical oxyde-1 pyridyle-4: selon la méthode décrite par R. A. Y. JONES et coll., J. Chem. Soc. 2937 (1960),

— lorsque R est un radical méthyl-6 oxyde-1 pyridazinyle-3: selon la méthode décrite dans le brevet belge 787 635.

— lorsque R est

1°) un radical dioxo-5,6 tétrahydro-1,4,5,6 triazine-1,2,4 yle-3 substitué en position -4 par un radical $R^{Y}$ choisi parmi:

a) un radical allyle, alcoyle (1 ou 2 atomes de carbone), lui-même éventuellement substitué par un radical alcoyloxy ou alcoylthio,

b) un radical dihydroxy-2,3 propyle ou dihydroxy-1,3 propyle-2 (éventuellement protégé sous forme d'acétal cyclique)

c) un radical alcoyle (2 ou 3 atomes de carbone) lui-même substitué [par hydroxy, carbamoyloxy, dialcoylamino, alcoylsulfonylamino, acylamino (éventuellement substitué), uréido, alcoyluréido ou dialcoyluréido]

d) un radical de formule générale (XXVIa) ou (XXVIb),

2°) un radical dioxo-5,6 tétrahydro-1,4,5,6 triazine-1,2,4 yle-3 substitué en position -1, ou dioxo-5,6 tétrahydro-1,2,5,6 triazine-1,2,4 yle-3 substitué en position -2 par un radical alcoyle contenant 1 ou 2 atomes de carbone ou par un radical de formule générale (XXVIa): en faisant agir un oxalate d'alcoyle sur une thiosemicarbazide de formules générales:

$$\left.\begin{array}{ll} R^{Y}\ NH\ CS\ NH{-}NH_2 & (a) \\ H_2N\ CS\ NH\ NH{-}R^{Y'} & (b) \\ H_2NCSN{-}NH_2 \text{ (N substitué par } R^{Y'}) & (c) \end{array}\right\} \quad (XXIX)$$

dans lesquelles $R^{Y}$ a la définition donnée ci-dessus en 1°) et $R^{Y'}$ est un substituant défini ci-dessus en 2°), en présence d'un alcoolate alcalin, par exemple l'éthylate ou le méthylate de sodium, ou le t.butylate de potassium, par application de la méthode décrite par M. PESSON et M. ANTOINE, Bull. Soc. Chim. France 1590 (1970).

Il n'est pas absolument nécessaire de purifier le produit obtenu (ni de libérer les radicaux protégés) pour le mettre en oeuvre pour la préparation des produits de formule générale (XVI).

La thiosemicarbazide de formule générale (XXIXa), (XXIXb) ou (XXIXc) peut être préparée selon l'une des méthodes décrites par K. A. JENSEN et coll., Acta Chem. Scand., *22*, 1 (1968), ou par application de la méthode déctrite par Y. KAZAKOV et J. Y. POTOVSKII, Doklady Acad. Nauk. SSSR, *134*, 824 (1960), étant entendu que, lorsque $R^{Y}$ contient un radical amino, ce dernier est protégé.

La protection du radical amino et l'élimination du radical protecteur s'effectuent selon les méthodes habituelles qui n'altèrent pas le reste de la molécule. On utilise notamment le groupement t-butoxycarbonyle, qui peut être éliminé par hydrolyse acide.

— Lorsque R est un radical alcoyl-1 triazol-1,3,4 yl-5: par application de l'une des méthodes décrites par M. PESSON et M. ANTOINE, Bull. Soc. Chim. France 1590 (1970).

— Lorsque R est un radical dioxo-5,6 tétrahydro-1,4,5,6 triazine-1,2,4 yle-3 substitué en position -4 par acyloxyalcoyle (éventuellement substitué): par acylation de la dioxo-5,6 hydroxyalcoyl-4 mercapto-3 tétrahydro-1,4,5,6 triazine-1,2,4 dont le radical mercapto a été préalablement protégé [par exemple selon C. G. KRUSE et coll., Tet. Lett. 1725 (1976)], par toute méthode connue pour acyler un alcool sans toucher au reste de la molécule, puis libération du groupement mercapto en milieu acide.

— Lorsque R est un radical alcoyl-2 dioxo-5,6 tétrahydro-1,2,5,6 triazine-1,2,4 yle-3, alcoyl-1 triazol-1,2,4 yle-5 ou alcoyl-1 alcoyloxycarbonyl-3 triazol-1,2,4 yle-5: selon la méthode décrite par M. PESSODN et M. ANTOINE, C. R. Acad. Sci., Série C, *267* (25), 1726 (1968).

— Lorsque R est un radical alcoyl-2 dihydro-2,5 oxo-5 triazine-1,2,4 yle-3 substitué en position -6 par un radical alcoyle ou alcoyloxy: selon la méthode décrite dans J. Antibiotics, *33*, 783 (1980).

— Lorsque R est un radical amino-1 dihydro-1,2 oxo-2 pyrimidinyle-4: selon la méthode décrite dans la demande de brevet européen EP 00005.

— Lorsque R est un radical thiadiazol-1,3,4 yle-5 éventuellement substitué par alcoyle: selon les méthodes décrites dans le brevet belge 830 821.

— Lorsque R est un radical thiadiazol-1,3,4 yle-5 substitué par dialcoylaminoalcoyl: selon la méthode décrite dans la demande de brevet allemand 2 446 254.

— Lorsque R est un radical thiadiazol-1,3,4 yle-5 substitué par un radical acylaminoalcoyle: selon la méthode décrite dans la demande de brevet japonais 76 80857.

— Lorsque R est un radical tétrazolyle-5 éventuellement substitué en position -1 par alcoyle ou hydroxyalcoyle: selon les méthodes décrites dans le brevet belge 830 821.

— Lorsque R est un radical tétrazolyle-5 substitué en position -1 par un radical dialcoylaminoalcoyle: par application de la méthode décrite dans la demande de brevet allemand 2 738 711.

— Lorsque R est un radical tétrazolyle-5 substitué par un radical acylaminoalcoyle: selon la méthode décrite dans le brevet US 4 117 123.

— Lorsque R est un radical tétrazolyle-5 substitué en position -1 par un radical dihydroxy-2,3 propyle: selon la méthode décrite dans le brevet US 4 064 242.

— Lorsque R est un radical tétrazolyle-5 substitué en position -1 par un radical dihydroxy-1,3 propyle-2: par addition d'azotura de sodium sur un isothiocyanate de diméthyl-2,2 dioxolanne-1,3 yle-5 (suivie éventuellement de la libération des groupements hydroxy).

— Lorsque R est un radical tétrazolyle-5 substitué en position -1 par un radical de formule générale (XXVIa): par action d'azoture de sodium sur l'isothiocyanate correspondant, par analogie avec la méthode décrite par R. E. ORTH, J. Pharm. Sci. *52* (9), 909 (1963).

Les isomères des produits de formules générales (I), (XII), (XIII), (XVI), (XVII), (XVIII), (XVIIIa), (XXII), (XXIII), (XXVII), (XXVIIa), (XXVIII) et (XXVIIIa) peuvent être séparés par chromatographie ou cristallisation.

Les dérivés de l'oxacéphalosporine de formule générale (XVI) et leurs sels pharmaceutiquement acceptables présentent des propriétés anti-bactériennes particulièrement intéressantes. Ils manifestent une activité remarquable in vitro et in vivo sur les germes Gram-positifs et Gram-négatifs.

In vitro, les produits de formule générale (XVI) se sont montrés actifs à une concentration comprise entre 1 et 15 µg/cm3 sur des suches de staphylocoques sensibles à la pénicilline G (Straphylococcus aureus Smith), à une concentration comprise entre 0,01 et 1 µg/cm3 sur Escherichia coli souche NIHJ.

In vivo les produits de formule générale (XVI) se sont montrés actifs sur les infections expérimentales de la souris à Staphylococcus aureus Smith (sensible à la pénicilline G) à une dose comprise entre 0,5 et 15 mg/kg par jour par voie sous-cutanée, et à Escherichia coli (souche NIHJ) à des doses comprises entre 0,01 et 10 mg/kg par jour par voie sous-cutanée.

Par ailleurs, la $DL_{50}$ des produits de formule générale (XVI) est comprise entre 1,5 g/kg et des doses supérieures à 2,5 g/kg par voie sous-cutanée chez la souris.

D'un intérêt particulier sont les produits de formule générale (I) dans laquelle

$R_1$ représente un radical protecteur d'acide choisi parmi benzhydryle, benzyle, méthoxyméthyle, p.méthoxybenzyle, p. nitrobenzyle et t.butyle,

$R_2$ représente un radical de formule générale (II) ou un radical protecteur d'amine facilement éliminable tel que défini en 2 a, b, c, et

$R_3$ et $R_4$ représentent des radicaux alcoyle et, plus spécialement, les produits de formule générale (I) dans laquelle

$R_1$ représente un radical benzhydryle ou p.nitrobenzyle,

$R_2$ représente un radical phénoxyacétyle, phénylacétyle, alcoyloxycarbonyle, trityle ou (tritylamino-2 thiazolyl-4)-2 méthoxyimino-2 acétyle

$R_3$ et $R_4$ représentent des radicaux méthyle,

R'' représente un atome d'hydrogène ou un radical méthoxy en position 7α, et en particulier la forme E des:

— benzhydryloxycarbonyl-2 (diméthylamino-2 vinyl)-3 oxo-8 tritylamino-7 oxa-5 aza-1 bicyclo [4.2.o] octène-2,

— benzhydryloxycarbonyl-2 (diméthylamino-2 vinyl)-3 oxo-8 phénylacétamido-7 oxa-5 aza-1 bicyclo [4.2.0] octène-2,

— benzhydryloxycarbonyl-2 (diméthylamino-2 vinyl)-3 méthoxy-7α oxo-8 phénoxyacétamido-7β oxa-5 aza-1 bicyclo [4.2.0] octène-2

— benzhydryloxycarbonyl-2 (diméthylamino-2 vinyl)-3 [méthoxyimino-2 (tritylamino-2 thiazolyl-4)-2 acétamido]-7 oxo-8 oxa-5 aza-1 bicycyclo [4.2.0] octène-2, isomère syn.

Les exemples suivants montrent comment l'invention peut être mise en pratique.

Dans ces exemples, les produits sont cités selon la nomenclature des Chemical Abstracts. Il est entendu qu'en l'absence de mention particulière les dérivés d'oxacéphalosporine qui sont cités présentent la stéréochimie donnée par la formule générale partielle:

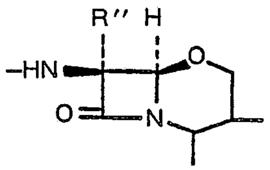

(XXX)

dans laquelle R'' est en position 7α.

Exemple 1

On chauffe à 80°C, sous azote, une solution de 4,25 g de benzhydryloxycarbonyl-2 méthyl-3 oxo-8 tritylamino-7 oxa-5 aza-1 bicyclo[4.2.0]octène-2 dans 20 cm3 de diméthylformamide. On ajoute, goutte à goutte en 7 minutes, dans la solution maintenue sous agitation à 80°C, 1,55 cm3 de t.butoxy bis-diméthylaminométhane. L'addition terminée, le mélange est conservé sous agitation à 80°C pendant 17 minutes. La solution est diluée par 150 cm3 d'acétate d'éthyle, la phase organique est lavée par trois fois 60 cm3 d'eau distillée et 60 cm3 d'une solution demi-saturée de chlorure de sodium, séchée sur sulfate de sodium, filtrée et concentrée à sec à 30°C sous pression réduite (20 mm de mercure; 2,7 kPa). Le résidu est trituré dans 150 cm3 d'éther éthylique, la suspension obtenue est filtrée et le filtrat est concentré à sec à 30°C sous pression réduite (20 mm de mercure; 2,7 kPa). On obtient 3,14 g d'un produit brut constitué principalement de benzhydryloxycarbonyl-2 (diméthylamino-2 vinyl)-3 oxo-8 tritylamino-7 oxa-5 aza-1 bicyclo[4.2.0] octène-2, forme E utilisable sans purification supplémentaire.

Spectre infra-rouge (KBr), bandes caractéristiques ($cm^{-1}$): 1780, 1660, 1615, 1490, 1450, 745, 700.

Spectre de RMN du proton (350 MHz, $CDCl_3$, δ en ppm, J en Hz): 2,77 (s, 6H, —$N(CH_3)_2$); 3,71 (d, J = 3,5, 1H, H en 6); 4,12 et 4,53 (2d, J = 17, 2H, —$CH_2$—O—); 4,26 (mf, 1H, H en 7); 6,24 et 6,40 (2d, J = 13, 2H, —CH=CH—); 6,81 (s, 1H, —COO—$CH(C_6H_5)_2$).

7,74 g de benzhydryloxycarbonyl-2 méthyl-3 oxo-8 tritylamino-7 oxa-5 aza-1 bicyclo[4.2.0] octène-2 sont préparés selon un schéma de synthèse décrit dans le brevet américain 4 108 992 dans lequel on remplace le glyoxylate de t-butyle par le glyoxylate de benzhydryle préparé selon le brevet français 1 495 047.

L'oxacéphalosporine attendue est obenue sous la forme d'un solide blanc à partir de 13,2 g de tritylamino-3 (propyne-2-yloxy)-4 oxo-2 azétidine.

Spectre infra-rouge (KBr), bandes caractéristiques ($cm^{-1}$): 3340, 1780, 1715, 1620, 1595, 1585, 1490, 1450, 1220, 745, 700.

Spectre de RMN du proton (350 MHz, $CDCl_3$, δ en ppm, J en Hz): 1,90 (s, 3H, —$CH_3$); 3,75 (d, J = 3,5, 1H, H en 6); 3,87 et 4,08 (2d, J = 18, 2H, —$CH_2$—O—); 4,30 (d, J = 3,5, 1H, H en 7); 6,85 (s, 1H, —COO—$CH(C_6H_5)_2$); 7,15 à 7,4 (mt, 26H, aromatiques et —HN—$C(C_6H_5)_3$).

Exemple 2

On chauffe à 80°C sous azote, une solution de 0,79 g de benzhydryloxycarbony6l-2 [méthoxyimino-2 (tritylamino-2 thiazolyl-4)-2 acétamido]-7 méthyl-3 oxo-8 oxa-5 aza-1 bicyclo [4.2.0] octène-2, isomère syn, dans 5 cm3 de diméthylformamide. On ajoute goutte à goutte en 6 minutes, dans la solution maintenue sous agitation à 80°C, 0,2 cm3 de t.butoxy bis-diméthylaminométhane. L'addition terminée, le mélange est conservé sous agitation à 80°C pendant 20 minutes. La solution est diluée par 25 cm3 d'acétate d'éthyle, la phase organique est lavée par trois fois 25 cm3 d'eau distillée et 25 cm3 d'une solution saturée de chlorure de sodium, séchée sur sulfate de magnésium, filtrée et concentrée à sec à 40°C sous pression réduite (20 mm de mercure; 2,7 kPa). On obtient 0,71 g d'un produit brut constitué principalement de benzhydryloxycarbonyl-2 (diméthylamino-2 vinyl)-3 [méthoxyimino-2 (tritylamino-2 thiazolyl-4)-2 acétamido]-7 oxo-8 oxa-5 aza-1 bicyclo [4.2.0] octène-2, isomère syn, forme E.

Un échantillon (0,4 g) est purifié par chromatographie sur une colonne de silice (0,04—0,06) (diamètre de la colonne: 2,2 cm; hauteur: 20 cm) en éluant sous 50 kPa avec un mélange de cyclohexane et d'acétate d'éthyle (40—60 en volumes) et en recueillant des fractions de 40 cm3. Les fractions 9 à 15 sont rassemblées et concentrées à sec sous pression réduite (30 mm de mercure; 4 kPa) à 40°C. On obtient 0,020 g d'une poudre jaune du produit pur.

Spectre de masse : pic moléculaire = 844

Spectre de RMN du proton (350 MHz, $CDCl_3$, δ en ppm, J en Hz) 2,86 (s, 6H, —$N(CH_3)_2$); 4,07 (s, 3H, =$NOCH_3$); 4,61 et 4,76 (2D, J = 18, 2H, —$CH_2O$—); 5,10 (D, J = 3,5, 1H, H en 6); 5,69 (DD, J = 3,5 et 9, 1H, H en 7); 6,36 et 6,54 (2D, J = 14, 2H, —CH=CH—); 6,67 (D, J = 9, 1H, —CONH—); 6.82 (D, 1H, H thiazole); 6,86 (s, 1H, —$CO_2CH(C_6H_5)_2$); 7,01 (s, 1H, —NH $C(C_6H_5)_3$); 7,15 à 7,65 (m, 25H)

Spectre infra-rouge (KBr), bandes caractéristiques ($cm^{-1}$) 1780, 1685, 1615, 1525, 1490, 1445, 1120, 1030, 740, 695.

Le benzhydryloxycarbonyl-2 [méthoxyimino-2 (tritylamino-2 thiazolyl-4)-2 acétamido]-7 méthyl-3 oxo-8 oxa-5 aza-1 bicyclo [4.2.0] octène-2, isomère syn, peut être préparé de la manière suivante:

Une solution de 35 g de tosylate d'amino-7 benzhydryloxycarbonyl-2 méthyl-3 oxo-8 oxa-5 aza-1 bicyclo [4.2.0] octène-2 dans 50 cm3 d'acétate d'éthyle est lavée par 15 cm3 d'une solution 1N de bicarbonate de sodium et 50 cm3 d'eau distillée puis séchée sur sulfate de magnésium. Après filtration, le

solvant est évaporé sous pression réduite (20 mm de mercure; 2,7 kPa) à 40°C. On obtient 2,33 g d'amino-7 benzhydryloxycarbonyl-2 méthyl-3 oxo-8 oxa-5 aza-1 bicyclo [4.2.0] octène-2 sous forme d'une meringue jaune pâle.

Spectre de masse: pic moléculaire = 364

Spectre infra-rouge (KBr), bandes caractéristiques ($cm^{-1}$) 3400, 3340, 1780, 1720, 1640, 1495, 1445, 1230, 1110, 1060, 745, 700.

Spectre de RMN du proton (350 MHz, $CDCl_3$, δ en ppm, J en Hz) 2,03 (s, 3H, $-CH_3$); 4,32 (s, 2H, $-CH_2O-$); 4,47 (D, J = 3,5, 1H, —H en 7); 4,92 (D, J = 3,5, 1H, —H en 6); 6,90 (s, 1H, $-CO_2C\underline{H}(C_6H_5)_2$); 7,15 à 7,45 (m, 10H aromatiques).

A une solution de 2,3 g d'amino-7 benzhydryloxycarbonyl-2 méthyl-3 oxo-8 oxa-5 aza-1 bicyclo [4.2.0] octène-2 et 2,79 g d'acide méthoxyimino-2 (tritylamino-2 thiazolyl-4)-2 acétique, isomère syn, dans 50 cm3 de chlorure de méthylène, on ajoute 1,56 g de N,N'-dicyclohexylcarbodiimide et 0,001 g de N,N-diméthylamino-4 pyridine en solution dans 40 cm3 de chlorure de méthylène en 15 minutes. Le mélange est maintenu sous agitation à 0°C pendant 2 heures. Il est alors transféré dans une ampoule à décanter, puis lavé successivement par 50 cm3 d'une solution 0,1 d'acide chlorhydrique, 50 cm3 d'eau distillée, 50 cm3 d'une solution demi-saturée de bicarbonate de sodium puis 50 cm3 d'eau distillée. La phase chlorométhylénique est séchée sur sulfate de magnésium anhydre. Le séchant filtré, la solution est concentrée à sec sous pression réduite (100 mm de mercure; 13,5 kPa). Le résidu (5,6 g) est purifié par chromatographie sur une colonne de silice (0,04—0,06) (diamètre de la colonne: 5,6 cm; hauteur: 26 cm) en éluant sous 50 kPa avec un mélange de cyclohexane et d'acétate d'éhyle (50—50 en volumes) et en recueillant des fractions de 120 cm3. Les fractions 8 à 20 sont rassemblées et concentrées à sec sous pression réduite (30 mm de mercure; 4 kPa) à 40°C. On obtient 4,02 g d'une meringue beige du benzhydryloxycarbonyl-2 [méthoxyimino-2 (tritylamino-2 thiazolyl-4)-2 acétamido]-7 méthyl-3 oxo-8 oxa-5 aza-1 bicyclo [4.2.0] octène-2, isomère syn, pur.

Spectre de masse: pic moléculaire = 789

Spectre infra-rouge (KBr), bandes caractéristiques ($cm^{-1}$): 2820, 1790, 1745, 1580, 1530, 1495, 1450, 1165, 1040, 750, 700

Spectre de RMN du proton (350 MHz, $CDCl_3$, δ en ppm, J en Hz) 2,04 (s, 3H, $-CH_3$); 4,07 (s, 3H, $=N-OCH_3$); 4,32 (s, 2H, $-CH_2O-$); 5,12 (D, J = 3,5, 1H, —H en 6); 5,75 (DD, J = 3,5 et 9, 1H, —H en 7); 6,68 (D, J = 9, 1H, —CONH—); 6,79 (s, 1H, H thiazole); 6,89 (s, 1H, $-CO_2C\underline{H}(C_6H_5)_2$); 7,00 (s large, 1H, $-N\underline{H}C(C_6H_5)_3$); 7,15 à 7,55 (m, 25 H aromatiques).

Le tosylate d'amino-7 benzhydryloxycarbonyl-2 méthyl-3 oxo-8 oxa-5 aza-1 bicyclo [4.2.0] octène-2 peut être obtenu de la manière suivante:

On agite à 40°C pendant 30 minutes une solution de 6,07 g de benzhydryloxycarbonyl-2 méthyl-3 oxo-8 tritylamino-7 oxa-5 aza-1 bicyclo [4.2.0] octène-2 et 1,9 g d'acide p.toluènesulfonique hydraté dans 100 cm3 d'acétone. Le mélange est refroidi à 0°C et un solide blanc précipite. On essore et on lave le gâteau par deux fois 5 cm3 d'acétone. On obtient ainsi 3,17 g de tosylate d'amino-7 benzhydryloxycarbonyl-2 méthyl-3 oxo-8 oxa-5 aza-1 bicyclo [4.2.0] octène-2.

Le filtrat acétonique est concentré à sec sous pression réduite (20 mm de mercure; 2,7 kPa). Le résidu est trituré dans deux fois 100 cm3 d'éther éthylique. L'éther est décanté et le solide obtenu est repris dans 20 cm3 d'acétate d'éthyle. On filtre et on obtient une deuxième fraction de 0,72 g de tosylate d'amino-7 benzhydryloxycarbonyl-2 méthyl-3 oxo-8 oxa-5 aza-1 bicyclo [4.2.0] octène-2.

Spectre infra-rouge ($CHBr_3$), bandes caractéristiques ($cm^{-1}$) 3300, 2400, 1800, 1720, 1500, 1455, 1225, 820, 760, 745, 570.

Spectre de RMN du proton (350 MHz, $CDCl_3$, δ en ppm, J en Hz) 1,73 (s, 3H, $-CH_3$); 2,15 (s, 3H, $-CH_3$ (APTS)); 3,98 (D, J = 19, 1H, $-CH_2O-$); 4,11 (D, J = 19, 1H, $-CH_2O-$); 4,78 (D, J = 2,5, 1H, —H en 7); 4,86 (D, J = 2,5, 1H, —H en 6); 6,87 (s, 1H, $-COOC\underline{H}(C_6H_5)_2$); 6,96 (D, J = 7,5, 1H, —H en ortho du méthyle, APTS); 7,10 à 7,60 (Mt, 10H aromatiques); 7,74 (D, J = 7,5, 1H, —H en ortho du $SO_3H$, APTS); 8,60 (Mf, 3H, $-NH_3^+$).

Exemple 3

On chauffe à 80°C sous azote, une solution de 0,527 g de benzhydryloxycarbonyl-2 méthyl-3 oxo-8 phénylacétamido-7 oxa-5 aza-1 bicyclo [4.2.0] octène-2 dans 5 cm3 de diméthylformamide. On ajoute goutte à goutte en 4 minutes dans la solution maintenue sous agitation à 80°C, 0,25 cm3 de t.butoxy bis-diméthylaminométhane. L'addition terminée, le mélange est conservé sous agitation à 80°C pendant 20 minutes. La solution est diluée par 25 cm3 d'acétate d'éthyle, la phase organique est lavée par cinq fois 25 cm3 d'eau distillée et 25 cm3 d'une solution saturée de chlorure de sodium, séchée sur sulfate de magnésium, filtrée et concentrée à sec à 40°C sous pression réduite (20 mm de mercure; 2,7 kPa). On obtient 0,44 g d'un produit brut constitué principalement de benzhydryloxycarbonyl-2 (diméthylamino-2 vinyl)-3 oxo-8 phénylacétamido-7 oxa-5 aza-1 bicyclo [4.2.0] octène-2, forme E.

On chromatographie 0,4 g de ce produit sur une colonne de silice (0,04—0,06) (diamètre de la colonne: 3,9 cm; hauteur: 24 cm) en éluant sous 50 kPa avec un mélange de cyclohexane et d'acétate d'éthyle (40—60 en volumes) et en receuillant des fractions de 25 cm3. Les fractions 25 à 30 sont rassemblées et concentrées à sec sous pression réduite (30 mm de mercure; 4 kPa) à 40°C. On obtient 0,030 g d'une meringue jaune se présentant comme un mélange (63—37 moles) de benzhydryloxycarbonyl-2 (diméthylamino-2 vinyl)-3 oxo-8 phénylacétamido-7 oxa-5 aza-1 bicyclo [4.2.0] octène-2 forme E et du dérivé (oxo-2 éthyl)-3 correspondant.

Caractéristiques physiques du benzhydryloxycarbonyl-2 (diméthylamino-2 vinyl)-3 oxo-8 phénylacétamido-7 oxa-5 aza-1 bicyclo [4.2.0] octène-2, forme E.

Spectre de masse: pic moléculaire = 537

Spectre infra-rouge (KBr); bandes caractéristiques ($cm^{-1}$) 3430, 1785, 1725, 1690, 1620, 1540, 1500, 1460, 1440, 1220, 1180, 1100, 760, 745, 705.

Spectre de RMN du proton (350 MHz, $CDCl_3$, δ en ppm, J en Hz) 2,82 (s, 6H, $—N(CH_3)_2$); 3,65 (s, 2H, $C_6H_5—CH_2—$); 4,49 (D, J = 16, 1H, $—CH_2—O—$); 4,62 (D, J = 16, 1H, $—CH_2—O—$); 4,98 (D, J = 3,5, 1H, —H en 6); 5,62 (DD, J = 9 et 3,5, 1H, —H en 7); 6,28 (D, J = 9, 1H, —CONH—); 6,31 (D, J = 13,5, 1H, —CH=CH—); 6,45 (D, J = 13,5, 1H, —CH=CH—); 6,85 (s, 1H, $—COOCH (C_6H_5)_2$); 7,10 à 7,60 (Mt, 15H aromatiques).

Le benzhydryloxycarbonyl-2 méthyl-3 oxo-8 phénylacétamido-7 oxa-5 aza-1 bicyclo [4.2.0] octène-2 peut être obtenu de la manière suivante:

On ajoute en 3 minutes une solution de 0,31 g de chlorure de phénylacétyle dans 1 cm3 de chlorure de méthylène anhydre à un mélange de 0,73 g d'amino-7 benzhydryloxycarbonyl-2 méthyl-3 oxo-8 oxa-5 aza-1 bicyclo [4.2.0] octène-2 et de 0,2 g de triéthylamine dans 20 cm3 de chlorure de méthylène anhydre maintenu à −20°C. On agite à cette température pendant 15 minutes, puis le mélange est transféré dans une ampoule à décanter où la phase chlorométhylénique est lavée successivement par 25 cm3 d'une solution 0,1 N d'acide chlorhydrique, 25 cm3 d'une solution demi-saturée de bicarbonate de sodium et 25 cm3 d'eau distillée. Elle est séchée sur sulfate de magnésium anhydre. On filtre et lave le sulfate de magnésium par 5 cm3 de chlorure de méthylène et les solutions organiques sont concentrées à sec sous pression réduite (100 mm de mercure; 13,5 kPa). On obtient 0,73 g de benzhydryloxycarbonyl-2 méthyl-3 oxo-8 phénylacétamido-7 oxa-5 aza-1 bicyclo [4.2.0] octène-2 sous la forme d'une meringue blanche.

Spectre de masse: pic moléculaire = 482

Spectre infra-rouge (KBr), bandes caractéristiques ($cm^{-1}$) 3420, 3360, 1790, 1725, 1685, 1670, 1540, 1500, 1460, 1230, 750, 700.

Spectre de RMN du proton (350 MHz, $CDCl_3$, δ en ppm, J en Hz) 1,99 (s, 3H, $—CH_3$); 3,66 (s, 2H, $C_6H_5—CH_2—$); 4,17 (D, J = 17,5, 1H $—CH_2—O—$); 4,25 (D, J = 17,5, 1H, $—CH_2—O—$); 5,01 (D, J = 3,5, 1H, —H en 6); 5,68 (DD, J = 10 et 3,5, 1H, —H en 7); 6,16 (D, J = 10, 1H, —CONH—); 6,88 (s, 1H, $—COOCH (C_6H_5)_2$); 7,13 à 7,60 (Mt, 15 H aromatiques).

## Exemple 4

On chauffe à 80°C, sous azote, une solution de 1,29 g de benzhydryloxycarbonyl-2 t.butoxycarbonylamino-7 méthyl-3 oxo-8 oxa-5 aza-1 bicyclo [4.2.0] octène-2 dans 15 cm3 de diméthylformamide. On ajoute goutte à goutte en 5 minutes, dans la solution maintenue sous agitation à 80°C, 0,7 cm3 de t.butoxy bisdiiméthylaminométhane. L'addition terminée, le mélange est maintenu sous agitation pendant 20 minutes. La solution est diluée par 100 cm3 d'acétate d'éthyle, la phase organique est lavée par trois fois 50 cm3 d'eau distillée et 50 cm3 d'une solution saturée de chlorure de sodium, séchée sur sulfate de magnésium, filtrée et concentrée à sec à 30°C sous pression réduite (20 mm de mercure; 2,7 kPa). On obtient 1,10 g d'un produit brut constitué principalement de benzhydryloxycarbonyl-2 t.butoxycarbonylamino-7 (diméthylkamino-2 vinyl)-3 oxo-8 oxa-5 aza-1 bicyclo [4.2.0] octène-2, forme E.

On chromatographie 1,04 g de ce produit sur une colonne de silice (0,04—0,06) (diamètre de la colonne: 4,5 cm, hauteur: 25 cm) en éluant sous 50 kPa avec un mélange de cyclohexane et d'acétate d'éthyle (60—40 en volumes) et en recueillant des fractions de 50 cm3. Les fractions 7 à 10 sont rassemblées et concentrées à sec sous pression réduite (30 mm de mercure; 4 kPa) à 40°C. On obtient 0,4 g d'une meringue jaune se présentant comme un mélange (44—56 en moles) de benzhydryloxycarbonyl-2 t.butoxycarbonylamino-7 (diméthylamino-2 vinyl)-3 oxo-8 oxa-5 aza-1 bicyclo [4.2.0] octène-2, forme E et du produit (oxo-2 éthyl)-3 correspondant.

Caractéristiques physiques du benzhydryloxycarbonyl-2 t.butoxycarbonylamino-7 (diméthylamino-2 vinyl)-3 oxo-8 oxa-5 aza-1 bicyclo [4.2.0] octène-2, forme E.

Spectre de masse: pic moléculaire = 519

Spectre infra-rouge (KBr), bandes caractéristiques ($cm^{-1}$) 3400, 2980, 2925, 2850, 1785, 1715, 1615, 1515, 1500, 1455, 1245, 1175, 1100, 1070, 750, 700.

Spectre de RMN du proton (350 MHz, $CDCl_3$, δ en ppm, J en Hz) 1,47 (s, 9H, $—C(CH_3)_3$); 2,85 (s, 6H, $—N(CH_3)_2$); 4,61 (D, J = 16, 1H, $—CH_2O—$); 4,77 (D, J = 16, 1H, $—CH_2O—$); 5,10 (D, J = 3,5, 1H, —H en 6); 5,30 (D, J = 10,5, 1H, —CONH—); 5,38 (DD, J = 10,5 et 3,5, 1H, —H en 7); 6,36 (D, J = 14, 1H, —CH=CH—); 6,53 (D, J = 14, 1H, —CH=CH—); 6,88 (s, 1H, $—COOCH (C_6H_5)_2$); 7,14 à 7,70 (m, 10H aromatiques).

Le benzhydryloxycarbonyl-2 t.butoxycarbonylamino-7 méthyl-3 oxo-8 oxa-5 aza-1 bicyclo [4.2.0] octène-2 peut être obtenu de la manière suivante:

On agite pendant 4 heures à 20°C 1 g d'amino-7 benzhydryloxycarbonyl-2 méthyl-3 oxo-8 oxa-5 aza-1 bicyclo [4.2.0] octène-2, 0,45 g de bicarbonate de sodium, 0,65 g de pyrocarbonate de di t.butyle dans un mélange de 4 cm3 d'eau distillée et 8 cm3 de dioxanne. Le mélange est concentré sous pression réduite (20 mm de mercure; 2,7 kPa) jusqu'à un volume résiduel de 5 cm3. Le résidu est dilué dans 10 cm3 d'acétate d'éthyle, et la phase organique est lavée par 10 cm3 d'eau distillée et séchée sur sulfate de

magnésium. On filtre et le filtrat est évaporé sous pression réduite (20 mm de mercure; 2,7 kPa). On obtient 1,25 g de benzhydryloxycarbonyl-2 t.butoxycarbonylamino-7 méthyl-3 oxo-8 oxa-5 aza-1 bicyclo [4.2.0] octène-2 sous la forme d'une meringue jaune pâle.

Spectre de masse: pic moléculaire: 464

Spectre infra-rouge (KBr), bandes caractéristiques ($cm^{-1}$) 3380, 1790, 1720, 1640, 1515, 1500, 1220, 1170, 750, 700

Spectre de RMN du proton (350 MHz, $CDCl_3$, δ en ppm, J en Hz) 1,48 (s, 9H, $-C(CH_3)_3$); 2,04 (s, 3H, $-CH_3$); 4,32 (s, 2H, $-CH_2O-$); 5,05 (D, J = 3,5, 1H, —H en 6); 5.26 (D, J = 10,5, 1H, —CONH—); 5,43 (DD, J = 3,5 et 10,5, 1H, H en 7); 6,89 (s, 1H, $-CO_2C\underline{H}$ $(C_6H_5)_2$); 7,23 à 7,60 (m, 10H aromatiques).

Exemple 5

On chauffe à 80°C sous azote une solution de 0,86 g de benzhydryloxycarbonyl-2 méthyl-3 oxo-8 phénoxyacétamido-7 oxa-5 aza-1 bicyclo [4.2.0] octène-2 dans 13 cm3 de diméthylformamide. On ajoute goutte à goutte en 2 minutes, dans la solution maintenue sous agitation à 80°C, 0,44 cm3 de t.butoxy bis-diméthylaminométhane. L'addition terminée, le mélange est conservé sous agitation à 80°C pendant 20 minutes. La solution est diluée par 100 cm3 d'acétate d'éthyle, la phase organique est lavée par trois fois 50 cm3 d'eau distillée et 50 cm3 d'une solution saturée de chlorure de sodium, séchée sur sulfate de magnésium, filtrée et concentrée à sec à 40°C sous pression réduite (20 mm de mercure; 2,7 kPa). On obtient 0,93 g d'un produit brut constitué principalement de benzhydryloxycarbonyl-2 (diméthylamino-2 vinyl)-3 oxo-8 phénoxyacétamido-7 oxa-5 aza-1 bicyclo [4.2.0] octène-2, forme E.

On purifie 0,90 g de ce produit par chromatographie sur une colonne de silice (0,04—0,06) (diamètre de la colonne: 3,8 cm hauteur: 23 cm) en éluant sous 50 kPa avec un mélange de cyclohexane et d'acétate d'éthyle (40—60 en volumes) et en recueillant des fractions de 40 cm3. Les fractions 9 à 13 sont rassemblées et concentrées à sec sous pression réduite (30 mm de mercure; 4 kPa) à 40°C. On obtient 0,12 g d'une meringue jaune orangé du produit pur.

Spectre infra-rouge (KBr), bandes caractéristiques ($cm^{-1}$) 3420, 1785, 1720, 1690, 1615, 1600, 1590, 1525, 1495, 1455, 1440, 1240, 1105, 1070, 765.

Spectre de RMN du proton (250 MHz, $CDCl_3$, δ en ppm, J en Hz) 2,86 (s, 6h, $-N(CH_3)_2$); 4,56 (AB, J = 14, 2H, $C_6H_5-O-CH_2-$); 4,60 (D, J = 16, 1H, $-O-CH_2-$); 4,79 (D, J = 16, 1H, $-O-CH_2-$); 5,08 (D, J = 4, 1H, H en —6); 5,70 (DD, J = 9 et 4, 1H, —H en 7); 6,36 (D, J = 14, 1H, —CH=CH—); 6,54 (D, J = 14, 1H, —CH=CH—); 6,88 (s, 1H, $-COOC\underline{H}(C_6H_5)_2$); 6,93 (D, J = 7,5, 2H, $C_6H_5-O-CH_2-$, H ortho); 7,03 (T, J = 7,5, 1H, $C_6H_5$ $OCH_2-$, H para); 7,10 à 7,70 (Mt, aromatiques et —CONH)—).

Le benzhydryloxycarbonyl-2 méthyl-3 oxo-8 phénoxyacétamido-7 oxa-5 aza-1 bicyclo [4.2.0] octène-2 peut être préparé de la manière suivante:

On ajoute en 15 minutes une solution de 1,71 g de chlorure de phénoxyacétyle dans 5 cm3 de chlorure de méthylène anhydre dans un mélange de 3,64 g d'amino-7 benzhydryloxycarbonyl-2 méthyl-3 oxo-8 oxa-5 aza-1 bicyclo [4.2.0] octène-2 et de 1,01 g de triéthylamine dans 50 cm3 de chlorure de méthylène anhydre maintenu à −20°C. On agite à cette température pendant 15 minutes puis on enlève le bain réfrigérant et on laisse remonter la température du mélange vers 20°C. Le mélange est lavé successivement par 50 cm3 d'une solution d'acide chlorhydrique 0,1 N, 50 cm3 d'eau distillée, 50 cm3 d'une solution demi-saturée de bicarbonate de sodium, 50 cm3 d'eau distillée et 50 cm3 d'une solution demi-saturée de chlorure de sodium. La solution chlorométhylénique est alors séchée sur sulfate de magnésium anhydre, filtrée et concentrée à sec sous pression réduite (100 mm de mercure; 13,5 kPa). On obtient un résidu meringué (4,73 g) constitué de benzhydryloxycarbonyl-2 méthyl-3 oxo-8 phénoxyacétamido-7 oxa-5 aza-1 bicyclo [4.2.0] octène-2.

Spectre de masse: pic moléculaire = 498

Spectre infra-rouge (KBr), bandes caractéristiques ($cm^{-1}$) 3420, 3350, 1790, 1720, 1690, 1640, 1600, 1590, 1530, 1495, 1490, 1455, 1440, 1230, 1110, 1065, 755, 700.

Spectre de RMN du proton (350 MHz, $CDCl_3$, δ en ppm, J en Hz) 2,04 (s, 3H, $-CH_3$); 4,34 (s, 2H, $-CH_2O-$); 4,58 (s, 2H, $C_6H_5O-CH_2-$) 5,11 (D, J = 3,5, 1H, —H en 6); 5,75 (DD, J = 10,5 et 3,5, 1H, —H en 7); 6,92 (s, 1H, $-COOC\underline{H}(C_6H_5)_2$); 6,94 (D, J = 7,5H, 2H, $C_6$ $H_5O-$ (Hortho)); 7,04 (t, J = 7,5, 1H, $C_6H_5O-$ (H para)); 7,20 à 7,60 (Mt, 13H aromatiques et —CO—N$\underline{H}$—).

Exemple 6

On chauffe à 80°C sous azote, une solution de 1,5 g de benzhydryloxycarbonyl-2 méthoxy-7α méthyl-3 oxo-8 phénoxyacétamido-7 oxa-5 aza-1 bicyclo [4.2.0] octène-2 dans 0,7 cm3 de diméthylformamide. Dans la solution maintenue sous agitation à 80°C, on ajoute goutte à goutte en 5 minutes, 0,7 cm3 de t.butoxycarbonylbis- diméthylaminométhane. L'addition terminée, le mélange est conservé sous agitation à 80°C pendant 30 minutes. La solution est diluée par 100 cm3 d'acétate d'éthyle, la phase organique est lavée par six fois 100 cm3 d'eau distillée et 100 cm3 d'une solution saturée de chlorure de sodium, séchée sur sulfate de magnésium, filtrée et concentrée à sec sous pression réduite (20 mm de mercure; 2,7 kPa) à 40°C. On obtient 1,19 g d'un produit brut constitué principalement de benzhydryloxycarbonyl-2 (diméthylamino-2 vinyl)-3 méthoxy-7α oxo-8 phénoxyacétamido-7 oxa-5 aza-1 bicyclo [4.2.0] octène-2, forme E.

Spectre de masse: pic moléculaire = 583

Spectre infra-rouge (KBr), bandes caractéristiques ($cm^{-1}$) 1775, 1700, 1615, 1495, 1460, 1440, 760, 700

Spectre de RMN du proton (350 MHz, $CDCl_3$, δ en ppm, J en Hz) 2,85 (s, 6H, $—N(CH_3)_2$); 3,58 (s, 3H, $CH_3O—$); 4,57 (s, 2H, $—O—CH_2—$); 4,63 (s, 2H, $C_6H_5$ $OCH_2—$); 5,14 (s, 1H, —H en 6); 6,30 (D, J = 14, 1H; —CH=CH—); 6,53 (D, J = 14, 1H, —CH=CH—); 6,88 (s, 1H, $—COOCH(C_6H_5)_2$); 6,9 à 7,6 (Mt, 15H aromatiques)

Le benzhydryloxycarbonyl-2 méthoxy-7α méthyl-3 oxo-8 phénoxyacétamido-7 oxa-5 aza-1 bicyclo [4.2.0] octène-2 peut être obtenu de la manière suivante:

On ajoute 13 cm3 d'une solution 1,6 N de n.butyllithium dans l'hexane à 50 cm3 de tétrahydrofuranne sec refroidi à −40°C et maintenu sous azote. On ajoute lentement 3 cm3 de méthanol anhydre, puis on refroidit le mélange à −74°C et on ajoute une solution de 3 g de benzhydryloxycarbonyl-2 méthyl-3 oxo-8 phénoxyacétamido-7 oxa-5 aza-1 bicyclo [4.2.0] octène-2 dans 14 cm3 de tétrahydrofuranne. La solution obtenue est maintenue à −74°C pendant 3 minutes et on ajoute 0,84 cm3 d'hypochlorite de t.butyle. Le mélange est maintenu sous agitation à −74°C pendant 1 heure et 20 minutes, puis on verse en filet 2 cm3 d'un mélange d'acide acétique glacial et de triéthylphosphite (50—50 en volumes). On laisse la température du mélange réactionnel remonter vers 20° et on dilue par 100 cm3 d'acétate d'éthyle. La phase organique est lavée successivement par 100 cm3 d'une solution de bicarbonate de sodium à 5%, 100 cm3 d'eau distillée et 100 cm3 d'une solution saturée de chlorure de sodium, séchée sur sulfate de magnésium anhydre, filtrée et le solvant est évaporé sous 20 mm de mercure (2,7 kPa). Le résidu est purifié par chromatographie sur gel de silice (0,04—0,06) (diamètre de la colonne: 4,2 cm, hauteur de silice: 24 cm) en éluant par un mélange de cyclohexane et d'acétate d'éthyle (55—45 en volumes) sous une pression de 40 kPa et en recueillant des fractions de 50 cm3. Les fractions 10 à 17 sont concentrées à sec sous pression réduite (30 mm de mercure, 4 kPa) à 30°C. On obtient 1,55 g de benzhydryloxycarbonyl-2 méthoxy-7α méthyl-3 oxo-8 phénoxyacétamido-7 oxa-5 aza-1 bicyclo [4.2.0] octène-2 sous la forme d'une meringue blanche.

Spectre de masse: pic moléculaire = 528

Spectre infra-rouge ($CHBr_3$), bandes caractéristiques ($cm^{-1}$) 3410, 1780, 1710, 1645, 1600, 1590, 1500, 1460, 1440, 1230, 1080, 760.

Spectre de RMN du proton (350 MHz, $CDCl_3$, δ en ppm, J en Hz) 2,02 (s, 3H, $—CH_3$); 3,58 (s, 3H, $—OCH_3$); 4,27 (D, J = 17,5, 1H, $—OCH_2—$); 4,34 (D, J = 17,5, 1H, $—O—CH_2—$); 4,58 (s, 2H, $C_6H_5—O—CH_2—$); 5,14 (s, 1H, —H en 6); 6,92 (s, 1H, $—COOCH(C_6H_5)_2$); 6,96 (D, J = 7, 2H, $C_6H_5—O—$, H ortho); 7,05 (T, J = 7, 1H, H en para de $C_6H_5O—$; 7,15 à 7,60 (Mt, H aromatiques + —CONH—).

## Exemple 7

On chauffe à 70°C sous azote, une solution de 0,58 g de méthyl-3 p.nitrobenzyloxycarbonyl-2 oxo-8 tritylamino-7 oxa-5 aza-1 bicyclo [4.2.0] octène-2 dans 10 cm3 de dioxanne sec. On ajoute goutte à goutte en 10 minutes une solution de 0,5 cm3 de t.butoxy bis-diméthylaminométhane dans 2 cm3 de dioxanne sec. L'addition terminée, le mélange est conservé sous agitation à 70°C pendant 20 minutes. La solution est diluée par 100 cm3 d'acétate d'éthyle, la phase organique est lavée par six fois 100 cm3 d'une solution demi-saturée de chlorure de sodium, séchée sur sulfate de sodium, filtrée et concentrée à sec à 40°C sous pression réduite (20 mm de mercure; 2,7 kPa). On obtient 0,2 g d'un produit brut que l'on purifie par chromatographie sur une colonne de silice (0,04—0,06) (diamètre de la colonne: 2,1 cm, hauteur: 20 cm) en éluant sous 50 kPa avec un mélange de cyclohexane et d'acétate d'éthyle (60—40 en volumes) et en recueillant des fractions de 15 cm3. La fraction 9 est concentrée à sec sous pression réduite (30 mm de mercure, 4 kPa) à 40°C. On obtient 0,02 g d'une poudre jaune se présentant comme un mélange de (diméthylamino-2 vinyl)-3 p.nitrobenzyloxycarbonyl-2 oxo-8 tritylamino-7 oxa-5 aza-1 bicyclo [4.2.0] octène-2, forme E et du dérivé (oxo-2 éthyl)-3 correspondant.

Spectre de RMN du proton (250 MHz, $CDCl_3$, δ en ppm, J en Hz) 2,83 (s, 6H, $—N(CH_3)_2$); 3,75 (D, J = 3, 1H, —H en 6); 4,21 (D, J = 15,5, 1H, $—O—CH_2—$); 4,43 (DD, J = 9 et 3, 1H, —H en 7); 4,56 (D, J = 15,5, 1H, $—O—CH_2—$); 5,18 (D, J = 12,0; 1H, $—CH_2— C_6H_4NO_2$); 5,41 (D, J = 12,0, 1H, $—CH_2—C_6H_4NO_2$); 6,21 (D, J = 13,75, 1H, —CH=CH—); 6,48 (D, J = 13,75, 1H, —CH=CH—); 7,10 à 7,75 (Mt, 18H aromatiques et $(C_6H_5)_3$ C—NH—); 8,20 (D, J = 8, 2H aromatiques en ortho du nitro).

Les exemples de référence ci-après illustrent l'utilisation des produits selon l'invention.

## Exemple de Reference 1

Le benzhydryloxycarbonyl-2 (diméthylamino-2 vinyl)-3 oxo-8 tritylamino-7 oxa-5 aza-1 bicyclo [4.2.0] octène-2 peut être mis en oeuvre de la manière suivante:

Une solution de 3,0 g de benzhydryloxycarbonyl-2 (diméthylamino-2 vinyl)-3 oxo-8 tritylamino-7 oxa-5 aza-1 bicyclo [4.2.0] octène-2, forme E, dans 100 cm3 d'acétate d'éthyle est agitée vigoureusement pendant 1 heure 30 à 20°C en présence de 45 cm3 d'une solution d'acide chlorhydrique 1N. Le mélange est décanté et la phase organique est lavée par 50 cm3 d'une solution à 5% de bicarbonate de sodium puis par 50 cm3 d'une solution demi-saturée de chlorure de sodium. La phase acétate d'éthyle est séchée sur sulfate de sodium, filtrée et concentrée à sec à 30°C sous pression réduite (20 mm de mercure; 2,7 kPa). On obtient 2,52 g d'un produit brut constitué principalement de benzhydryloxycarbonyl-2 (oxo-2 éthyl)-3 oxo-8 tritylamino-7 oxa-5 aza-1 bicyclo [4.2.0] octène-2, sous la forme d'une meringue marron clair.

Spectre infra-rouge (KBr), bandes caractéristiques ($cm^{-1}$) 1790, 1720, 1600, 1495, 1450, 1220, 750, 700.

Spectre de RMN du proton (350 MHz, $CDCl_3$, δ en ppm, J en Hz) 3,37 et 3,50

(2d, J = 16, 2H, 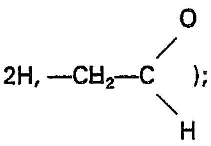 );

3,81 (d, J = 3,5, 1H, H en 6); 3,92 et 4,12 (2d, J = 18, 2H, —$CH_2$—O—); 4,35 (dd, J = 3,5 et 9, 1H, H en 7); 6,80 (s, 1H, —COO—$CH(C_6H_5)_2$); 9,49

(s, 1H, 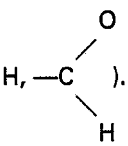 ).

On dissout 2,51 g de benzhydryloxycarbonyl-2 (oxo-2 éthyl)-3 oxo-8 tritylamino-7 oxa-5 aza-1 bicyclo [4.2.0] octène-2 dans 20 cm3 de pyridine. A la solution obtenue, on ajoute 1,13 g de chlorure de tosyle et on maintient le mélange sous agitation à 20°C pendant 1 heure 25 minutes. La solution est versée dans 150 cm3 d'eau glacée, une gomme se dépose sur les parois du récipient, la phase aqueuse est décantée et on dissout la substance gommeuse dans 45 cm3 d'acétate d'éthyle. La solution organique est lavée par 2 fois 50 cm3 d'une solution 0,1 N d'acide chlorhydrique, 50 cm3 d'une solution à 5% de bicarbonate de sodium et 30 cm3 d'une solution demi-saturée de chlorure de sodium, séchée sur sulfate de sodium, filtrée et concentrée à sec à 30°C sous pression réduite (20 mm de mercure; 2,7 kPa). On recueille 2,4 g d'un produit brut marron constitué essentiellement de benzhydryloxycarbonyl-2 oxo-8 (tosyloxy-2 vinyl)-3 tritylamino-7 oxa-5 aza-1 bicyclo [4.2.0] octène-2, mélange des formes E et Z.

Spectre infra-rouge (KBr), bandes caractéristiques ($cm^{-1}$) 1790, 1725, 1595, 1490, 1450, 1380, 1190, 1180, 745, 700.

A une solution de 2,4 g de benzhydryloxycarbonyl-2 oxo-8 (tosyloxy-2 vinyl)-3 tritylamino-7 oxa-5 aza-1 bicyclo [4.2.0] octène-2 mélange des formes E et Z, dans 15 cm3 de diméthylformamide, on ajoute 0,28 g de sel de sodium de mercapto-5 méthyl-2 thiadiazole-1,3,4. Le mélange est agité à 20°C pendant 2 heures, puis est dilué par 50 cm3 d'acétate d'éthyle. La solution est lavée par 5 fois 50 cm3 d'eau distillée et 50 cm3 d'une solution demi-saturée de chlorure de sodium, puis séchée sur sulfate de sodium. On filtre et concentre à sec à 20°C sous pression réduite (20 mm de mercure; 2,7 kPa). Le résidu (2,1 g) est chromatographié sur une colonne de gel de silice Merck (0,04—0,06) (diamètre de la colonne: 4,1 cm, hauteur: 20 cm). On élue par 1 litre d'un mélange de cyclohexane-acétate d'éthyle 70—30 (en volumes) sous une pression de 50 kPa en recueillant des fractions de 60 cm3. Les fractions 9 et 10 sont concentrées à sec sous pression réduite (30 mm de mercure; 4 kPa) à 30°C. On obtient 0,13 g de benzhydryloxycarbonyl-2 [(méthyl-2 thiadiazol-1,3,4 yl-5) thio-2 vinyl]-3 oxo-8 tritylamino-7 oxa-5 aza-1 bicyclo [4.2.0] octène-2, forme E sous la forme d'une meringue jaune pâle.

Spectre infra-rouge (KBre), bandes caractéristiques ($cm^{-1}$): 1790, 1720, 1490, 1450, 1210, 745, 700.

Spectre de RMN du proton (350 MHz, $CDCL_3$, δ en ppm J en Hz) 2,74 (s, 3H, —$CH_3$); 3,76 (d, J = 3,5, 1H, H en 6); 4,16 et 4,62 (2d, J = 18, 2H, —$CH_2$—O—); 4,37 (D, J = 3,5, 1H, H en 7); 6,84 (s, 1H, —COO—$CH(C_6H_5)_2$); 6,96 (d, J = 17, 1H, —CH=CH—S—).

On dissout 0,112 g de benzhydryloxycarbonyl-2 [(méthyl-2 thiadiazol-1,3,4 yl-5) thio-2 vinyl]-3 oxo-8 tritylamino-7 oxa-5 aza-1 bicyclo [4.2.0] octène-2, forme E dans 1,4 cm3 d'acétone et on ajoute à la solution 0,029 g d'acide p.toluènesulfonique monohydraté. La solution est portée au reflux pendant 45 minutes pendant lesquelles des cristaux se développent sur les parois du récipient. La suspension est filtrée et le filtrat est versé dans 10 cm3 d'une solution à 1% de bicarbonate de sodium. Le mélange est extrait par 2 fois 5 cm3 d'acétate d'éthyle, la phase organique est séchée sur sulfate de sodium, filtrée et concentrée à sec à 30°C sous pression réduite (20 mm de mercure; 2,7 kPa). On recueille ainsi 0,1 g d'un solide brut marron constitué essentiellement d'amino-7 benzhydryloxycarbonyl-2 [(méthyl-2 thiadiazol-1,3,4 yl-5) thio-2 vinyl]-3 oxo-8 oxa-5 aza-1 bicyclo [4.2.0] octène, forme E.

Rf = 0,20 [chromatoplaque de silicagel, éluant: cyclohexane-acétate d'éthyle 80—20 (en volumes)].

A une solution de 0,1 g d'amino-7 benzhydryloxycarbonyl-2 [(méthyl-2 thiadiazol-1,3,4 yl-5) thio-2 vinyl]-3 oxo-8 oxa-5 aza-1 bicyclo [4.2.0] octène-2, forme E dans 20 cm3 de chlorure de méthylène, on ajoute 0,089 g d'acide méthoxyimino-2 (tritylamino-2 thiazolyl-4)-2 acétique, isomère syn, 0,041 g de N,N'-dicyclohexylcarbodiimide et 0,001 g de N,N-diméthylamino-4 pyridine. Le mélange est agité à 20°C

pendant 1 heure 30. On ajoute alors 0,1 cm3 d'acide acétique; on élimine un léger insoluble par filtration, concentre à sec le filtrat à 30°C sous pression réduite (100 mm de mercure; 13,3 kPa) et dissout le résidu dans 5 cm3 d'acétate d'éthyle. La solution est lavée par 2 fois 2,5 cm3 d'acide chlorhydrique 0,1 N, puis par 5 cm3 d'une solution à 1% de bicarbonate de sodium et par 5 cm3 d'eau distillée. On sèche sur sulfate de sodium, filtre et concentre à sec à 30°C (20 mm de mercure; 2,7 kPa). Le résidu (0,12 g) est fixé sur 0,25 g de gel de silice Merck (0,05—0,2) et la poudre obtenue est déposée sur une colonne de 10 g de gel de silice (diamètre de la colonne: 1 cm). On élue successivement par 70 cm3 d'un mélange cyclohexane-acétate d'éthyle 50—50 (en volumes), 30 cm3 d'un mélange 40—60 et 30 cm3 d'un mélange 20—80 en recueillant des fractions de 2,5 cm3. On concentre à sec à 20°C sous pression réduite (20 mm de mercure; 2,7 kPa) les fractions 22 à 40 et recueille 0,023 g de benzhydryloxycarbonyl-2 [méthoxyimino-2 (tritylamino-2 thiazolyl-4)-2 acétamido]-7 [(méthyl-2 thiadiazol-1,3,4 yl-5) thio-2 vinyl]-3 oxo-8 oxa-5 aza-1 bicyclo [4.2.0] octène-2 isomère syn, forme E sous la forme d'une meringue orangée.

Spectre infra-rouge (KBr), bandes caractéristiques ($cm^{-1}$) 1795, 1720, 1685, 1520, 1495, 1450, 1210, 1045, 750, 700.

Spectre de RMN du proton (350 MHz, $CDCl_3$, δ en ppm, J en Hz) 2,74 (s, 3H, $—CH_3$); 4,07 (s, 3H, $=N—O—CH_3$); 4,59 et 4,86 (2d, J = 18, 2H, $—CH_2—O—$); 5,13 (d, J = 3,5, 1H, —H en 6); 5,81 (dd, J = 3,5 et 9, 1H, —H en 7); 6,78 (s, 1H, —H du thiazole); 6,79 (d, J = 9, 1H, —CO—NH—); 6,88 (s, 1H, —COO—CH $(C_6H_5)_2$); 7,03 (mf, 1H, $—NH—C(C_6H_5)_3$); 7,14 et 7,69 (2d, J = 17, 2H, —CH=CH—S—).

A une solution de 0,023 g de benzhydryloxycarbonyl-2 [méthoxyimino-2 (tritylamino-2 thiazolyl-4)-2 acétamido]-7 [(méthyl-2 thiadiazol-1,3,4 yl-5) thio-2 vinyl]-3 oxo-8 oxa-5 aza-1 bicyclo [4.2.0] octène-2, isomère syn, forme E, dans 1 cm3 d'acide formique, on ajoute 0,5 cm3 d'eau distillée et chauffe le mélange à 50°C sous agitation pendant 20 minutes. Après refroidissement, on filtre l'insoluble et on concentre à sec à 30°C sous pression réduite (20 mm de mercure; 2,7 kPa). On reprend le résidu par 2 fois 15 cm3 d'éthanol en concentrant à sec à chaque fois à 20°C sous 20 mm de mercure (2,7 kPa) et triture le résidu dans 20 cm3 d'éther éthylique. On obtient après filtration 0,010 g d'[(amino-2 thiazolyl-4)-2 méthoxyimino-2 acétamido]-7 carboxy-2 [(méthyl-2 thiadiazol-1,3,4 yl-5) thio-2 vinyl]-3 oxo-8 oxa-5 aza·1 bicyclo [4.2.0] octène-2, isomère syn, forme E sous la forme d'une poudre jaune.

Spectre infra-rouge (KBr), bandes caractéristiques ($cm^{-1}$) 1785, 1670, 1620, 1530, 1040.

Spectre de RMN du proton (350 MHz, DMSO $d_6$, δ en ppm, J en Hz) 2,70 (s, $—CH_3$); 3,83 (s, $=N—OCH_3$); 4,50 et 4,78 (2d, J = 18, $—CH_2—O—$); 5,14 (d, J = 3,5, —H en 6); 5,45 (mf, —H en 7); 6,75 (s, —H du thiazole); 7,10 à 7,70 (mt, $—NH_2$ et —CH=CH—S—); 9,34 (d, J = 9, —CO—NH).

## Exemple de Reference 2

Le produit de l'exemple 2 peut être utilisé de la manière suivante:

Une solution de 1,05 g de benzhydryloxycarbonyl-2 (diméthylamino-2 vinyl)-3 [méthoxyimino-2 (tritylamino-2 thiazolyl-4)-2 acétamido]-7 oxo-8 oxa-5 aza-1 bicyclo [4.2.0] octène-2, isomère syn, forme E dans 10 cm3 d'acétate d'éthyle est agitée vigoureusement pendant 1 heure à 20°C en présence de 5 cm3 d'une solution 1N d'acide chlorhydrique. Le mélange est décanté et la phase organique est lavée par 10 cm3 d'une solution saturée de bicarbonate de sodium, 10 cm3 d'eau distillée et 10 cm3 d'une solution saturée de chlorure de sodium. La phase acétate d'éthyle est séchée sur sulfate de magnésium anhydre, filtrée et concentrée à sec à 30°C sous pression réduite (20 mm de mercure; 2,7 kPa). On obtient 1,00 g de benzhydryloxycarbonyl-2 [méthoxyimino-2 (tritylamino-2 thiazolyl-4)-2 acétamido]-7 oxo-8 (oxo-2 éthyl)-3 oxa-5 aza-1 bicyclo [4.2.0] octène-2, isomère syn sous la forme d'une meringue jaune.

Spectre infra-rouge (KBr), bandes caractéristiques ($cm^{-1}$) 3400, 1795, 1725, 1685, 1525, 1495, 1450, 1035, 755, 700.

Spectre de masse: pic moléculaire = 817

Spectre de RMN du proton (250 MHz, $CDCl_3$, δ en ppm, J en Hz) 3,52 (D, J = 16,25, 1H, $—CH_2CHO$); 3,62 (D, J = 16,25, 1H, $—CH_2—CHO$); 4,07 (s, 3H, $=NOCH_3$); 4,35 (s, 2H, $—CH_2O—$); 5,17 (D, J = 3,75, 1H, —H en 6); 5.82 (DD, J = 10 et 3,75, 1H, —H en 7); 6,76 (D, J = 10, 1H, —CONH—); 6.80 (s, 1H, —H du thiazole); 6,85 (s, 1H, $—COOCH(C_6H_5)_2$; 7,13 (Mf, 1H, $(C_6H_5)_3$ CNH—); 7,20 à 7,55 (Mf, 25H, aromatiques); 9,60 (s, 1H, —CHo).

On ajoute en 10 minutes 0,27 cm3 de triéthylamine à une solution refroidie à −5°C de 1,00 g de benzhydryloxycarbonyl-2 [méthoxyimino-2 (tritylamino-2 thiazolyl-4)-2 acétamido]-7 oxo-8 (oxo-2 éthyl)-3 oxa-5 aza-1 bicyclo [4.2.0] octène-2, isomère syn, et de 0,35 g de chlorure de tosyle dans 20 cm3 de dichlorométhane. On laisse sous agitation à −5°C pendant 10 minutes puis on laisse remonter à 20°C. Le mélange est alors agité pendant 30 minutes. Le solvant est évaporé sous pression réduite (350 mm de mercure, 47 kPa). On obtient ainsi une huile marron qui se solidifie par agitation avec 100 cm3 d'éther. Le solide est séparé sur filtre, et séché. On obtient 0,38 g de benzhydryloxycarbonyl-2 [méthoxyimino-2 (tritylamino-2 thiazolyl-4)-2 acétamido]-7 oxo-8 (tosyloxy-2 vinyl)-3 oxa-5 aza-1 bicyclo [4.2.0] octène-2, isomère syn, forme E, sous la forme d'une poudre de couleur crème. Le filtrat est concentré jusqu'à un volume résiduel de 5 cm3, et on ajoute 100 cm3 d'éther isopropylique. Un solide jaune pâle se sépare. On l'isole sur filtre et obtient 0,11 g d'une deuxième fraction de benzhydryloxycarbonyl-2 [méthoxyimino-2 (tritylamino-2 thiazolyl-4)-2 acétamido]-7 oxo-8 (tosyloxy-2 vinyl)-3 oxa-5 aza-1 bicyclo [4.2.0] octène-2, isomère syn, forme E.

Spectre infra-rouge (KBr), bandes caractéristiques ($cm^{-1}$) 3400, 1800, 1720, 1685, 1600, 1520, 1495, 1450, 1380, 1195, 1180, 1070, 815, 755, 700.

Spectre de RMN du proton (250 MHz, $CDCl_3$) δ en ppm, J en Hz) 2,44 (s, 3H, $CH_3$ tosyl); 4,08 (s, 3H, $=NOCH_3$); 4,44 (D, J = 16,25, 1H, $-O-CH_2-$); 4,64 (D, J = 16,25, 1H, $-O-CH_2-$); 5,12 (D, J = 3,75, 1H, —H en 6); 5,79 (DD, J = 8 et 3,75, —1H, —H en 7); 6,72 (D, J = 8, 1H, —CONH—); 6,77 (s, 1H, —H du thiazole); 6,82 (D, J = 12,5, 1H, —CH=CH—); 6,83 (s, 1H, $-COOCH(C_6H_5)_2$); 7,03 (Mf, 1H, $(C_6H_5)_3$ CNH); 7,17 (D, J = 12,5, 1H, —CH=CH—); 7,20 à 7,55 (Mf, 27H aromatique); 7,75 (D, J = 8, 2H ortho du tosyle).

Une solution de 0,53 g de benzhydryloxycarbonyl-2 [méthoxyimino-2 (tritylamino-2 thiazolyl-4)-2 acétamido]-7 oxo-8 (tosyloxy-2 vinyl)-3 oxa-5 aza-1 bicyclo [4.2.0] octène-2, isomère syn, forme E, et de 0,139 g de [(diméthoxy-2,2 éthyl)-4 dioxo-5,6 tétrahydro-1,4,5,6 triazine-1,2,4 yl-3] thiolate de sodium dans 12 cm3 de diméthylformamide est portée à 40°C sous agitation pendant 6 heures. Le mélange est transféré dans une ampoule à décanter contenant 30 cm3 d'acétate d'éthyle et 50 cm3 d'eau distillée. Après décantation la phase organique est lavée par 3 fois 50 cm3 d'eau distillée puis séchée sur sulfate de magnésium anhydre. Le solvant est évaporé sous pression réduite (20 mm de mercure; 2,7 kPa) et le résidu (0,52 g) est purifié par chromatographie sur une colonne de gel de silice Merck (0,04—0,06) (diamètre de la colonne: 2,2 cm, hauteur: 25,5 cm). On élue par un mélange de cyclohexane-acétate d'éthyle 15—85 (en volumes) sous une pression de 50 kPa en recueillant des fractions de 20 cm3. Les fractions 10 à 28 sont concentrées à sec sous pression réduite (30 mm de mercure; 4 kPa) à 30°C. On obtient 0,20 g de benzhydryloxycarbonyl-2 {[(diméthoxy-2,2 éthyl)-4 dioxo-5,6 tétrahydro-1,4,5,6 triazine-1,2,4 yl-3] thio-2 vinyl}-3 [méthoxyimino-2 (tritylamino-2 thiazolyl-4)-2 acétamido]-7 oxo-8 oxa-5 aza-1 bicyclo [4.2.0] octène-2, isomère syn, forme E, sous la forme d'une meringue marron clair.

Spectre de RMN de proton (250 MHz, $CDCl_3$, δ en ppm, J en Hz) 3,42 (s, 6H, $(-OCH_3)$); 4,03 (AB limite, J = 4,5 et 10, 2H, $>NCH_2-$); 4,06 (s, 3H, $=N-OCH_3$); 4,58 (D, J = 16,25, 1H, $-OCH_2-$); 4,65 (T, J=5, 1H, $-CH(OCH_3)_2$); 4,84 (D, J=16,25, 1H, $-O-CH_2-$); 5,15, (D, J = 3,15, 1H, —H en 6); 5,81 (DD, J = 10 et 3,15, 1H, —H en 7); 6,73 (D, J = 16,25, 1H, —CH=CH—); 6,79 (s, 1H, —H du thiazole); 6,87 (s, 1H, —COO $CH(C_6H_5)_2$); 7,05 (Mf, 1H, $(C_6H_5)_3$ CNH; 7,18 (D, J = 10, 1H, —CONH—); 7,20 à 7,55 (Mf, 25H aromatiques); 7,68 (D, J = 16,25, 1H, —CH=CH—); 10,09 (s large, 1H, —NH— triazine).

On ajoute 0,74 g d'acide p.toluènesulfonique monohydraté à une solution de 0,20 g de benzhydryloxycarbonyl-2 {[(diméthoxy-2,2 éthyl)-4 dioxo-5,6 tétrahydro-1,4,5,6 triazine-1,2,4 yl-3] thio-2 vinyl}-3 [méthoxyimino-2 (tritylamino-2 thiazolyl-4)-2 acétamido]-7 oxo-8 oxa-5 aza-1 bicyclo [4.2.0] octène 2, isomère syn, forme E, dans 5 cm3 d'acétonitrile à 50°C. Le mélange est maintenu à cette température pendant 30 minutes. Après refroidissement à 20°C, on filtre le précipité qui s'est développé au cours de la réaction.

Il est lavé par 1 cm3 d'acétonitrile, puis il est agité vigoureusement dans 5 cm3 d'eau distillée pendant 30 minutes. La suspension est filtrée, et la poudre marron obtenue est séchée sous pression réduite (10 mm de mercure; 1,33 kPa) donnant 20 mg de tosylate d'[amino-2 thiazolyl-4)-2 méthoxyimino-2 acétamido]-7 carboxy-2 {[dioxo-5,6 (oxo-2 éthyl)-4 tétrahydro-1,4,5,6 triazine-1,2,4 yl-3] thio-2 vinyl}-3 oxo-8 oxa-5 aza-1 bicyclo [4.2.0] octène-2, isomère syn, forme E.

Spectre de RMN du proton (250 MHz, $CF_3CO_2D$, δ en ppm, J en Hz) 2,47 (s, 3H, $-CH_3$); 4,30 (s, 3H, $=NOCH_3$); 5,17 (Mf, $>NCH_2-$); 5,45 (Mf, 1H, —H en 6); 5,91 (Mf, 1H, —H en 7); 7,39 (D, J = 8, 2H, H tosyle); 7,53 (s, 1H, —H du thiazole); 7,82 (D, J = 8, 2H, H tosyl); 9,76 (s, 1H, —CHO).

## Exemple de Reference 3

Le mélange (63/37 moles) obtenu après chromatographie à l'exemple 3 peut être mis en oeuvre de la manière suivante:

Une solution de 0,02 g du mélange dans 5 cm3 d'acétate d'éthyle est agitée vigoureusement pendant 30 minutes à 20°C en présence de 3 cm3 d'une solution d'acide chlorhydrique 1N. Le mélangé est décanté et la phase organique est lavée par 10 cm3 d'eau distillée, 10 cm3 d'une solution à 5% de bicarbonate de sodium, 10 cm3 d'eau distillée et 10 cm3 d'une solution demi-saturée de chlorure de sodium. La phase acétate d'éthyle est séchée sur sulfate de magnésium anhydre, filtrée et concentrée à sec à 30°C sous pression réduite (20 mm de mercure; 2,7 kPa). On obtient 0,02 g de benzhydryloxycarbonyl-2 (oxo-2 éthyl)-3 oxo-8 phénylacétamido-7 oxa-5 aza-1 bicyclo [4.2.0] octène-2, sous la forme d'une poudre jaune clair.

Spectre de masse: pic moléculaire = 510.

Spectre infra-rouge (KBr), bandes caractéristiques ($cm^{-1}$) 3420, 3370, 2930, 2860, 2740, 1792, 1720, 1685, 1670, 1650, 1530, 1500, 1460, 1230, 1110, 1070, 760, 750, 705.

Spectre de RMN du proton (350 MHz, $CDCl_3$, δ en ppm, J en Hz) 3,48 (D, J = 17,5, 1HJ, $-CH_2CHO$); 3,54 (D, J = 17,5, 1H, $-CH_2CHO$); 3,65 (s, 2H, $C_6H_5-CH_2-$); 4,22 (AB limite, J ≃ 16, 2H, $-CH_2-O-$); 5,05 (D, J = 3,5, 1H, —H en 6); 5,74 (DD, J = 10 et 3,5, 1H, —H en 7); 6,28 (D, J = 10, 1H, —CO—NH—); 6,84 (s, 1H, $-COOCH (C_6H_5)_2$); 7,10 à 7,60 (Mt, 15H aromatiques); 9,53 (s, 1H, —CHO).

## Exemple de Reference 4

Le mélange obtenu après chromatographie à l'exemple 4 peut être mis en oeuvre de la manière suivante:

Une solution de 0,16 g du mélange dans 5 cm3 d'acétate d'éthyle est agitée vigoureusement pendant 30 minutes à 20°C en présence de 3 cm3 d'une solution d'acide chlorhydrique 1N. Le mélange est décanté

et la phase organique est lavée par 5 cm3 d'une solution à 5% de bicarbonate de sodium, 5 cm3 d'eau distillée, puis 5 cm3 d'une solution demi-saturée de chlorure de sodium. La phase acétate d'éthyle est séchée sur sulfate de magnésium anhydre, filtrée et concentrée à sec à 30°C sous pression réduite (20 mm de mercure; 2,7 kPa). On obtient 0,11 g de benzhydryloxycarbonyl-2 t. butoxycarbonylamino-7 (oxo-2 éthyl)-3 oxo-8 oxa-5 aza-1 bicyclo [4.2.0] octène-2, sous la forme d'une meringue marron clair.

Spectre de masse: pic moléculaire = 492.

Spectre infra-rouge (KBr), bandes caractéristiques ($cm^{-1}$) 1790, 1720, 1515, 1500 +1455, 1390, 1370, 1165, 750, 700

Spectre de RMN du proton (350 MHz, $CDCl_3$, δ en ppm, J en Hz) 1,47 (s, 9H, —$C(CH_3)_3$); 3,52 (D, J = 16, 1H, —$CH_2$—CHO); 3,62 (D, J = 16, 1H, —$CH_2$—CHO); 4,36 (AB limite, J ≃ 17,5, 2H, —$CH_2$—O—); 5,10 (D, J = 3,5, 1H, —H en 6); 5,27 (D, J = 10,5, 1H, —CONH); 5,47 (DD, J = 10,5 et 3,5, 1H, —H en 7); 6,87 (s, 1H, —COOCH $(C_6H_5)_2$); 7,20 à 7,60 (Mt, 10H aromatiques); 9,58 (s, 1H, —CHO).

Exemple de Reference 5

Le produit de l'exemple 5 peut être mis en oeuvre de la manière suivante:

Une solution de 0,09 g de benzhydryloxycarbonyl-2 (diméthylamino-2 vinyl)-3 oxo-8 phénoxyacétamido-7 oxa-5 aza-1 bicyclo [4.2.0] octène-2, forme E, dans 5 cm3 d'acétate d'éthyle est agitée vigoureusement pendant 30 minutes à 20°C en présence de 3 cm3 d'une solution d'acide chlorhydrique 1N. Le mélange est décanté et la phase organique est lavée par 10 cm3 d'eau distillée, 10 cm3 d'une solution à 5% de bicarbonate de sodium, 10 cm3 d'eau distillée puis 10 cm3 d'une solution demi-saturée de chlorure de sodium. La phase acétate d'éthyle est séchée sur sulfate de magnésium anhydre, filtrée et concentrée à sec à 30°C sous pression réduite (20 mm de mercure; 2,7 kPa). On obtient 0,07 g de benzhydryloxycarbonyl-2 (oxo-2 éthyl)-3 oxo-8 phénoxyacétamido-7 oxa-5 aza-1 bicyclo [4.2.0] octène-2 sous la forme d'une laque jaune.

Spectre de masse: pic moléculaire = 526

Spectre infra-rouge (KBr), bandes caractéristiques ($cm^{-1}$) 3420, 2930, 2860, 2730, 1790, 1720, 1690, 1670, 1650, 1530, 1495, 1455, 1440, 1240, 1110, 1070, 760, 700.

Spectre de RMN du proton (350 MHz, $CDCl_3$, δ en ppm, J en Hz) 3,52 (D, J = 17,5, 1H, —$CH_2$—CHO); 3,63 (d, J = 17,5, 1H, —$CH_2$—CHO); 4,32 (d, J = 17,5, 1H, —$CH_2$—O—); 4,40 (D, J = 17,5, 1H, —$CH_2$—O—); 4,58 (s, 2H, $C_6H_5$ O—$CH_2$—); 5,15 (D, J = 3,5, 1H, —H en 6); 5,80 (DD, J = 10 et 3,5, 1H, —H en 7); 6,88 (s, 1H, —$COOCH(C_6H_5)_2$; 6,9 à 7,60 (Mt, 16H aromatiques et —CONH—); 9,60 (s, 1H, —CHO).

Exemple de Reference 6

Le produit de l'exemple 6 peut être utilisé de la manière suivante:

Une solution de 0,010 g de benzhydryloxycarbonyl-2 (diméthylamino-2 vinyl)-3 méthoxy-7α oxo-8 phénoxyacétamido-7 oxa-5 aza-1 bicyclo [4.2.0] octène-2, forme E, dans 5 cm3 d'acétate d'éthyle est agitée vigoureusement pendant 30 minutes à 20°C en présence de 3 cm3 d'une solution d'acide chlorhydrique 1N. Le mélange est décanté et la phase organique est lavée par 10 cm3 d'eau distillée, 10 cm3 d'une solution saturée de bicarbonate de sodium, 10 cm3 d'eau distillée et 10 cm3 d'une solution saturée de chlorure de sodium. La phase acétate d'éthyle est séchée sur sulfate de magnésium anhydre, filtrée et concentrée à sec à 30°C sous pression réduite (20 mm de mercure; 2,7 kPa). On obtient 0,009 g de benzhydryloxycarbonyl-2 méthoxy-7α (oxo-2 éthyl)-3 oxo-8 phénoxyacétamido-7 oxa-5 aza-1 bicyclo [4.2.0] octène-2, sous la forme d'une meringue marron clair.

Spectre de masse: pic moléculaire = 556

Spectre de RMN du proton (350 MHz, $CDCl_3$ δ en ppm, J en Hz) 3,50 (s, —$OCH_3$); 4,25 (s, —O—$CH_2$—); 4,50 (s, $C_6H_5$ O—$CH_2$—); 5,08 (s, $H_6$); 6,79 (s, —$COOCH(C_6H_5)_2$); 9,48 (s, —CHo).

Exemple de Reference 7

Le mélange obtenu à l'exemple 7 peut être mis en oeuvre de la manière suivante:

Une solution de 0,016 g de ce mélange dans 5 cm3 d'acétate d'éthyle est agitée vigoureusement pendant 30 minutes à 20°C en présence de 5 cm3 d'une solution d'acide chlorhydrique 1N. Le mélange est décanté et la phase organique est lavée par 2 fois 5 cm3 d'une solution demi-saturée de bicarbonate de sodium puis par 2 fois 5 cm3 d'une solution demi-saturée de chlorure de sodium. La phase acétate d'éthyle est séchée sur sulfate de sodium anhydre, filtrée et concentrée à sec à 30°C sous pression réduite (20 mm de mercure; 2,7 kPa). On obtient 0,012 g de p.nitrobenzyloxycarbonyl-2 (oxo-2 éthyl)-3 oxo-8 tritylamino-7 oxa-5 aza-1 bicyclo [4.2.0] octène-2, sous la forme d'une meringue marron clair.

Spectre de RMN du proton (250 MHz, $CDCl_3$, δ en ppm, J en Hz) 3,44 (D, J = 16,25, 1H, —$CH_2$—CHO); 3,57 (D, J = 16,25, 1H, —$CH_2$—CHO); 3,84 (D, J = 3,75, 1H, —H en 6); 3,96 (D, J = 18,75, 1H, —$CH_2$—O—); 4,17 (D, J = 18,75, 1H, —$CH_2O$—); 4,34 (DD, J = 9 et 3,75, 1H, —H en 7); 5,21 (D, J = 12,0, 1H, —$CH_2C_6H_4NO_2$); 5,37 (D, J = 12,0, 1H, —$CH_2$ $C_6H_4NO_2$); 7,10 à 7,75 (Mt, 18H aromatiques et $(C_6H_5)_3$ C—NH—); 8,23 (D, J = 8, 2H aromatiques en ortho du nitro); 9,58 (s, 1H, —CHO).

En opérant par analogie avec la méthode décrite à l'exemple de référence 1, les produits des exemples de référence 3 à 7 peuvent être utilisés pour préparer une céphalosporine de formule générale (XVI).

**Revendications pour les Etats contractants: BE CH DE FR GB IT LI LU NL SE**

1. Un nouveau dérivé d'oxacéphalosporine caractérisé en ce qu'il répond à la formule générale:

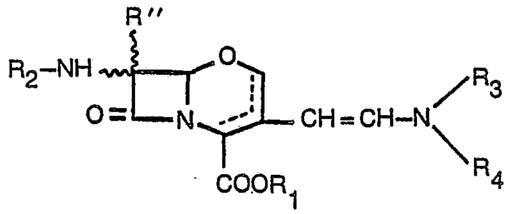

qui se présente sous forme bicyclooctène-2 ou -3, dans laquelle le substituant sur l'atome de carbone en position -3 du bicyclooctène présente la stéréoisomérie E ou Z, $R_1$ représente un radical protecteur d'acide facilement éliminable, $R_2$ représente soit un radical de formule générale:

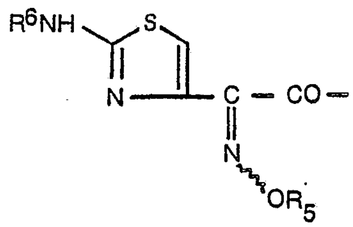

[dans laquelle $R_5$ est un atome d'hydrogène, un radical alcoyle, vinyle, carboxyalcoyle de formule générale:

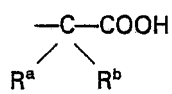

{dans laquelle $R^a$ et $R^b$ sont identiques ou différents et représentent des atomes d'hydrogène ou des radicaux alcoyle, ou forment ensemble un radical alcoylène contenant 2 ou 3 atomes de carbone}, et dont la fonction acide est protégée, ou un radical protecteur d'oxime, et $R^6$ est un radical protecteur d'amine], soit un radical α-carboxyarylacétyle dont la fonction acide est protégée et dans lequel aryle représente phényle, p.hydroxyphényle, p.hydroxyphényle protégé ou thiényle-2 ou -3, et R'' représente un atome d'hydrogène ou un radical méthoxy en position 7α, ou bien $R_2$ représente un radical facilement éliminable choisi parmi:

1) benzhydryle ou trityle

2) un radical acyle de formule générale:

$$R_7CO—$$

dans laquelle $R_7$ représente:

a) un atome d'hydrogène, un radical alcoyle contenant 1 à 7 atomes de carbone, méthyle substitué par 1 à 3 atomes d'halogène, alcényle contenant 3 à 7 atomes de carbone, ou cyanométhyle,

b) un radical phényle (pouvant être jusqu'à 3 fois substitué par des groupes hydroxy, nitro, cyano, trifluorométhyle, alcoyle ou alcoyloxy) ou un radical thiényle-2 ou -3,

c) un radical de formule générale:

$$R'_7—Y—CH_2—$$

dans laquelle $R'_7$ est un radical tel que défini en b) et Y est un atome de soufre ou d'oxygène,

d) un radical arylalcoyle de formule générale:

$$R''_7CH_2—$$

dans laquelle $R''_7$ est un radical phényle pouvant être jusqu'à 3 fois substitué (par des radicaux hydroxy, alcoyle ou alcoyloxy) ou un radical hétérocyclyle choisi parmi thiényle-2 ou -3, furyle-2 ou -3 et tétrazolyle-1

3) un radical de formule générale:

$$R_8OCO—$$

dans laquelle $R_8$ est un radical alcoyle ramifié non substitué, un radical alcoyle droit ou ramifié portant un ou plusieurs substituants choisis parmi les atomes d'halogène ou des radicaux cyano, phényle phényle substitué (par un ou plusieurs atomes d'halogène ou radicaux alcoyle, alcoyloxy, nitro ou phényle), un radical triméthylsilyl-2 éthyle, un radical vinyle ou allyle ou un radical quinolyle,

4) un radical de formule générale:

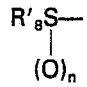 ou $R'_8Se$—

dans lesquelles $R'_8$ représente un reste alcoyle, phényle ou phényle substitué (par un ou plusieurs atomes d'halogène ou radicaux nitro ou alcoyle) et n est égal à 0 ou 1,

5) ou bien $R_2NH$— est remplacé par un radical diméthylaminométhylèneamino ou par un radical de formule générale:

$$Ar—CH=N—$$

dans laquelle Ar est un groupe phényle éventuellement substitué par un ou plusieurs radicaux méthoxy, nitro, alcoyle ou hydroxy, et R'' représente un atome d'hydrogène ou un radical méthoxy en position 7α, ou un atome d'hydrogène en position 7β, et $R_3$ et $R_4$ qui sont identiques ou différents représentent des radicaux alcoyle (éventuellement substitués par un radical alcoylоxy ou dialcoylamino) ou phényle, ou forment ensemble avec l'atome d'azote auquel ils sont rattachés un hétérocycle saturé à 5 ou 6 chaînons, contenant éventuellement un autre hétéroatome choisi parmi l'azote, l'oxygène ou le soufre et éventuellement substitué par un radical alcoyle, étant entendu que les radicaux alcoyle cités ci-dessus sont droits ou ramifiés et contiennent 1 à 4 atomes de carbone.

2. Un produit selon la revendication 1, caractérisé en ce que $R_1$ est un radical protecteur d'acide choisi parmi méthoxyméthyle, t.butyle, benzhydryle, benzyle, nitrobenzyle et p.méthoxybenzyle.

3. Procédé de préparation d'un produit selon la revendication 1, caractérisé en ce que l'on fait agir un produit de formule générale:

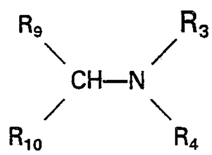

{dans laquelle $R_3$ et $R_4$ sont définis comme dans la revendication 1 et $R_9$ et $R_{10}$, qui sont identiques ou différents, soit représentent des groupements de formule générale

$$—X_2R_{11}$$

(dans laquelle $X_2$ est un atome d'oxygène et $R_{11}$ représente un radical alcoyle ou phényle) soit représentent l'un un radical $—X_2R_{11}$ (dans lequel $X_2$ est un atome d'oxygène ou de soufre et $R_{11}$ est défini comme ci-dessus) et l'autre un radical amino de formule générale

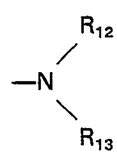

(dans laquelle $R_{12}$ et $R_{13}$ sont définis comme $R_3$ et $R_4$) soit encore représentent chacun un radical de formule générale $—NR_{12}R_{13}$}, sur un dérivé d'oxacéphalosporine de formule générale:

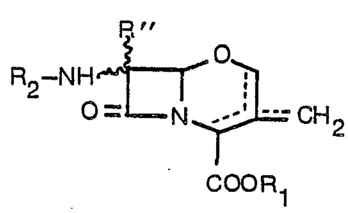

dans laquelle $R_1$, $R_2$ et R'' sont définis comme dans la revendication 1, et qui se présente sous forme bicyclooctène-2 ou -3 ou méthylène-3 bicyclooctane, puis on sépare éventuellement le produit obtenu en ses isomères.

**Revendication pour l'Etat contractant: AT**

Procédé de préparation d'un nouveau dérivé d'oxacéphalosporine de formule générale:

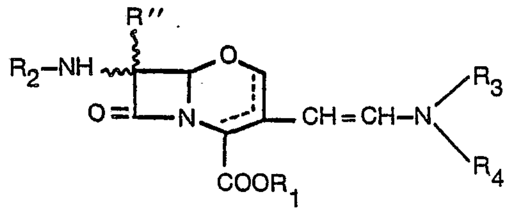

qui se présente sous forme bicyclooctène-2 ou -3, dans laquelle le substituant sur l'atome de carbone en position -3 du bicyclooctène présente la stéréoisomérie E ou Z, $R_1$ représente un radical protecteur d'acide facilement éliminable, $R_2$ représente soit un radical de formule générale:

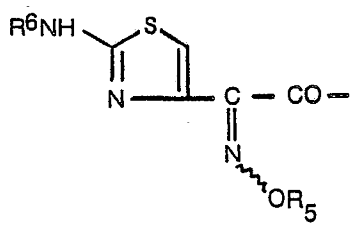

[dans laquelle $R_5$ est un atome d'hydrogène, un radical alcoyle, vinyle, carboxyalcoyle de formule générale:

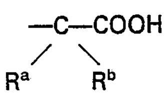

{dans laquelle $R^a$ et $R^b$ sont identiques ou différents et représentent des atomes d'hydrogène ou des radicaux alcoyle, ou forment ensemble un radical alcoylène contenant 2 ou 3 atomes de carbone}, et dont la fonction acide est protégée, ou un radical protecteur d'oxime, et $R^6$ est un radical protecteur d'amine], soit un radical α-carboxyarylacétyle dont la fonction acide est protégee et dans lequel aryle représente phényle, p.hydroxyphényle, p.hydroxyphényle protégé ou thiényle-2 ou -3, et R'' représente un atome d'hydrogène ou un radical méthoxy en position 7α, ou bien $R_2$ représente un radical protecteur facilement éliminable choisi parmi:

1) benzhydryle ou trityle
2) un radical acyle de formule générale:

$$R_7CO—$$

dans laquelle $R_7$ représente:

a) un atome d'hydrogène, un radical alcoyle contenant 1 à 7 atomes de carbone, méthyle substitué par 1 à 3 atomes d'halogène, alcényle contenant 3 à 7 atomes de carbone, ou cyanométhyle,

b) un radical phényle (pouvant être jusqu'à 3 fois substitué par des groupes hydroxy, nitro, cyano, trifluorométhyle, alcoyle ou alcoyloxy) ou un radical thiényle-2 ou -3,

c) un radical de formule générale:

$$R'_7—Y—CH_2—$$

dans laquelle $R'_7$ est un radical tel que défini en b) et Y est un atome de soufre ou d'oxygène,

d) un radical arylalçoyle de formule générale:

$$R''_7CH_2—$$

dans laquelle $R''_7$ est un radical phényle pouvant être jusqu'à 3 fois substitué (par des radicaux hydroxy, alcoyle ou alcoyloxy) ou un radical hétérocyclyle choici parmi thiényle-2 ou -3, furyle-2 ou -3 et tétrazolyle-1

3) un radical de formule générale:

$$R_8OCO—$$

dans laquelle $R_8$ est un radical alcoyle ramifié non substitué, un radical alcoyle droit ou ramifié portant un ou plusieurs substituants choisis parmi les atomes d'halogène ou des radicaux cyano, phényle phényle substitué (par un ou plusieurs atomes d'halogène ou radicaux alcoyle, alcoyloxy, nitro ou phényle), un radical triméthylsilyl-2 éthyle, un radical vinyle ou allyle ou un radical quinolyle,

4) un radical de formule générale:

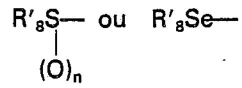

dans lesquelles $R'_8$ représente un reste alcoyle, phényle ou phényle substitué (par un ou plusieurs atomes d'halogène ou radicaux nitro ou alcoyle) et n est égal à 0 ou 1,

5) ou bien $R_2NH—$ est remplacé par un radical diméthylaminométhylèneamino ou par un radical de formule générale:

$$Ar—CH{=}N—$$

dans laquelle Ar est un groupe phényle éventuellement substitué par un ou plusieurs radicaux méthoxy, nitro, alcoyle ou hydroxy, R'' représente un atome d'hydrogène ou un radical méthoxy en position 7α, ou un atome d'hydrogène en position 7β, et $R_3$ et $R_4$ qui sont identiques ou différents représentent des radicaux alcoyle (éventuellement substitués par un radical alcoyloxy ou dialcoylamino) ou phényle, ou forment ensemble avec l'atome d'azote auquel ils sont rattachés un hétérocycle saturé à 5 ou 6 chaînons, contenant éventuellement un autre hétéroatome choici parmi l'azote, l'oxygène ou le soufre et éventuellement substitué par un radical alcoyle, étant entendu que les radicaux alcoyle cités ci-dessus sont droits ou ramifiés et contiennent 1 à 4 atomes de carbone, caractérisé en ce que l'on fait agir un produit de formule générale:

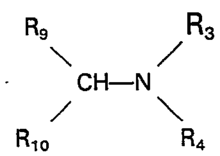

{dans laquelle $R_3$ et $R_4$ sont définis comme précédemment et $R_9$ et $R_{10}$, qui sont identiques ou différents, soit représentent des groupements de formule générale:

$$—X_2R_{11}$$

(dans laquelle $X_2$ est un atome d'oxygène et $R_{11}$ représente un radical alcoyle ou phényle) soit représentent l'un un radical $—X_2R_{11}$ (dans lequel $X_2$ est un atome d'oxygène ou de soufre et $R_{11}$ est défini comme ci-dessus) et l'autre un radical amino de formule générale

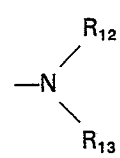

(dans laquelle $R_{12}$ et $R_{13}$ sont définis comme $R_3$ et $R_4$) soit encore représentent chacun un radical de formule générale $—NR_{12}R_{13}$}, sur un dérivé d'oxacéphalosporine de formule générale:

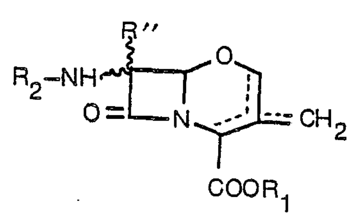

dans laquelle $R_1$, $R_2$ et R'' sont définis comme précédemment, et qui se présente sous forme bicyclooctène-2 ou -3 ou méthylène-3 bicyclooctane, puis on sépare éventuellement le produit obtenu en ses isomères.

**Patentansprüche für die Vertragsstaaten: BE CH LI DE FR GB IT LU NL SE**

1. Ein neues Oxacephalosporinderivat, dadurch gekennzeichnet, daß es der allgemeinen Formel

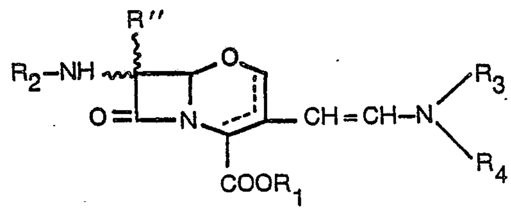

entspricht, die in der Bicycloocten-2- oder -3-Form vorliegt, worin der Substituent am Kohlenstoffatom in 3-Stellung des Bicyclooctens die E— oder Z—Stereoisomerie aufweist, $R_1$ einen leicht entfernbaren Säureschutzrest bedeutet, $R_2$ entweder einen Rest der allgemeinen Formel

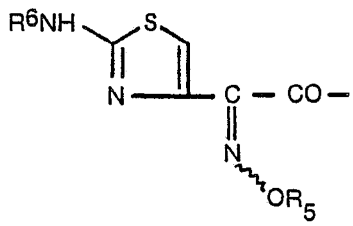

bedeutet [worin $R_5$ ein Wasserstoffatom, ein Alkyl-, Vinyl-, Carboxyalkylrest der allgemeinen Formel

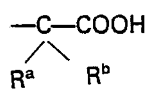

ist {worin $R^a$ und $R^b$ identisch oder verschieden sind und Wasserstoffatome oder Alkylreste bedeuten oder zusammen einen Alkylenrest mit 2 oder 3 Kohlenstoffatomen bilden} und dessen Säurefunktion geschützt ist, oder ein Oximschutzrest ist und $R^6$ ein Aminschutzrest ist], oder einen α-Carboxyarylacetylrest bedeutet, dessen Säurefunktion geschützt ist und worin Aryl, Phenyl, p-Hydroxyphenyl oder geschütztes p-Hydroxyphenyl oder Thienyl-2 oder -3 bedeutet, und R'' ein Wasserstoffatom oder einen Methoxyrest in 7α-Stellung darstellt oder aber $R_2$ bedeutet einen leicht entfernbaren Rest ausgewählt unter:

1) Benzhydryl oder Trityl
2) einem Acylrest der allgemeinen Formel

$$R_7CO—$$

worin $R_7$ bedeutet:

a) ein Wasserstoffatom, einen Alkylrest mit 1 bis 7 Kohlenstoffatomen, Methyl substituiert durch 1 bis 3 Halogenatome, Alkenyl enthaltend 3 bis 7 Kohlenstoffatome oder Cyanomethyl,

b) einen Phenylrest (der bis zu dreimal substituiert sein kann durch Hydroxy-, Nitro-, Cyano-, Trifluormethyl-, Alkyl- oder Alkyloxygruppen) oder einen Thienyl-2-oder -3-Rest,

c) einen Rest der allgemeinen Formel:

$$R'_7—Y—CH_2—$$

worin $R'_7$ ein Rest wie er unter b) definiert ist, ist und Y ein Schwefel- oder Sauerstoffatom darstellt,

d) einen Arylalkylrest der allgemeinen Formel:

$$R''_7CH_2—$$

worin $R''_7$ ein Phenylrest ist, der bis zu dreimal substituiert sein kann (durch Hydroxy-, Alkyl- oder Alkyloxyreste) oder ein heterocyclischen Rest ausgewählt unter Thienyl-2 oder -3, Furyl-2 oder -3 und Tetrazolyl-1,

3) einem Rest der allgemeinen Formel

$$R_8OCO—$$

worin $R_8$ ein verzweigter nicht-substituierter Alkylrest ist, ein gerader oder verzweigter Alkylrest mit einem oder mehreren Substituenten ausgewählt unter den Halogenatomen, oder Cyano-, Phenyl-, substituiertem Phenyl- (durch ein oder mehrere Halogenatome oder Alkyl-, Alkyloxy-, Nitro- oder Phenylreste), ein 2-Trimethylsilyläthylrest, ein Vinyl- oder Allylrest oder ein Chinolylrest,

4) einem Rest der allgemeinen Formel

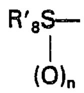 oder $R'_8Se—$

worin $R'_8$ einen Alkyl-, Phenyl- oder substituierten Phenylrest (durch ein oder mehrere Halogenatome oder Nitrooder Alkylreste) bedeutet und n gleich 0 oder 1 ist,

5) oder aber $R_2NH—$ ist durch einen Dimethylaminomethylenaminorest oder durch einen Rest der allgemeinen Formel

$$Ar—CH=N—$$

ersetzt, worin Ar eine Phenylgruppe ist, die gegebenenfalls durch einen oder mehrere Methoxy-, Nitro-, Alkyl- oder Hydroxyreste substituiert ist, und R'' bedeutet ein Wasserstoffatom oder einen Methoxyrest in 7α-Stellung oder ein Wasserstoffatom in 7β-Stellung und $R_3$ und $R_4$, welche identisch oder voneinander verschieden sind, bedeuten Alkylreste (gegebenenfalls substituiert durch einen Alkyloxy- oder Dialkylaminorest) oder Phenyl oder bilden zusammen mit dem Stickstoffatom, an das sie gebunden sind, einen gesättigten Heterocyclus mit 5 oder 6 Ringgliedern, enthaltend gegebenenfalls ein weiteres Heteroatom ausgewählt unter Stickstoff, Sauerstoff oder Schwefel und gegebenenfalls substituiert durch einen Alkylrest, wobei die oben erwähnten Alkylreste gerade oder verzweigt sind und 1 bis 4 Kohlenstoffatome enthalten.

2. Ein Produkt gemäß Anspruch 1, dadurch gekennzeichnet, daß $R_1$ ein Säureschutzrest ist ausgewählt unter Methoxymethyl, tert.-Butyl, Benzhydryl, Benzyl, Nitrobenzyl und p-Methoxybenzyl.

3. Verfahren zur Herstellung eines Produkts gemäß Anspruch 1, dadurch gekennzeichnet, daß man ein Produkt der allgemeinen Formel

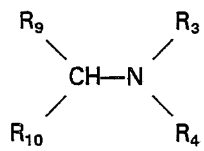

{worin $R_3$ und $R_4$ wie in Anspruch 1 definiert sind und $R_9$ und $R_{10}$, welche identische oder verschieden sind, entweder Gruppen der allgemeinen Formel

$$—X_2R_{11}$$

bedeuten, (worin $X_2$ ein Sauerstoffatom ist und $R_{11}$ einen Alkyl- oder Phenylrest bedeutet) oder eines einen Rest $—X_2R_{11}$ (worin $X_2$ ein Sauerstoffatom oder Schwefelatom ist $R_{11}$ wie oben definiert ist) und das andere einen Aminorest der allgemeinen Formel

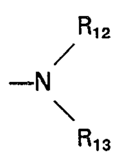

(worin $R_{12}$ und $R_{13}$ wie $R_3$ und $R_4$ definiert sind) bedeutet oder auch jedes bedeutet einen Rest der allgemeinen Formel $—NR_{12}R_{13}$} auf ein Oxacephalosporinderivat der allgemeinen Formel

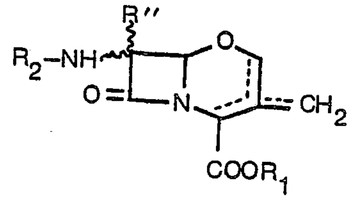

worin $R_1$, $R_2$ und R'' wie in Anspruch 1 definiert sind und das in Bicycloocten-2- oder -3-Form oder 3-Methylen-bicyclooctan-Form vorliet, einwirken läßt und daß man dann gegebenenfalls das erhaltene Produkt in seine Isomeren auftrennt.

**Patentansprüch für den Vertragsstaat: AT**

Verfahren zur Herstellung eines neuen Oxacephalosporinderivats der allgemeinen Formel:

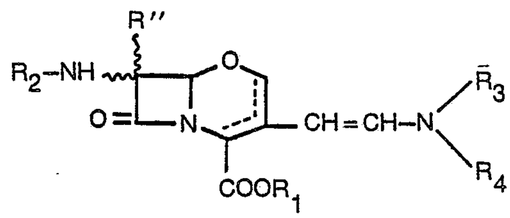

welchus in 2- oder 3-Bicycloocten-Form vorliegt, worin der Substituent am in 3-Stellung des Bicyclooctens befindlichen Kohlenstoffatom E— oder Z—Stereoisomerie aufweist, $R_1$ eine leicht entfernbare Säureschutzgruppe darstellt, $R_2$ entweder einen Rest der allgemeinen Formel:

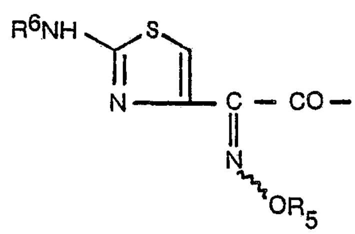

[worin $R_5$ ein Wasserstoffatom, ein Alkylrest, Vinylrest, Carboxyalkylrest der allgemeinen Formel:

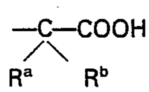

{in welcher $R^a$ und $R^b$ gleich oder verschieden sind und Wasserstoffatome oder Alkylrest darstellen oder zusammen einen Alkylenrest mit 2 oder 3 Kohlenstoffatomen bilden}, dessen Säurefunktion geschützt ist, oder eine Oximschutzgruppe ist und $R^6$ eine Aminschutzgruppe ist] oder einen α-Carboxyarylacetylrest, dessen Säurefunktion geschützt ist und worin Aryl Phenyl, p-Hydroxyphenyl, geschütztes p-Hydroxyphenyl oder 2- oder 3-Thienyl bedeutet und R'' ein Wasserstoffatom oder einen Methoxyrest in Position 7α darstellt, oder $R_2$ eine leicht entfernbare Schutzgruppe, ausgewählt aus:

1) Benzhydryl oder Trityl
2) einem Acylrest der allgemeinen Formel:

$R_7CO—$

worin $R_7$ darstellt:

a) ein Wasserstoffatom, einen Alkylrest mit 1 bis 7 Kohlenstoffatomen, Methyl, substituiert durch 1 bis 3 Halogenatome, Alkenyl mit 3 bis 7 Kohlenstoffatomen, oder Cyanomethyl,

b) einen Phenylrest (der bis zu dreimal durch Hydroxy-, Nitro-, Cyano-, Trifluormethyl-, Alkyl- oder Alkyloxygruppen substituiert sein kann) oder einen 2- oder 3-Thienylrest,

c) einen Rest der allgemeinen Formel:

$$R'_7—Y—CH_2—$$

worin $R'_7$ ein wie unter b) definierter Rest ist und Y ein Schwefeloder Sauerstoffatom ist,

d) einen Arylalkylrest der allgemeinen Formel:

$$R''_7CH_2—$$

worin $R''_7$ ein Phenylrest, der bis zu dreimal (durch Hydroxy-, Alkyl- oder Alkyloxyreste) substituiert sein kann, oder ein Heterocyclylrest, ausgewählt aus 2- oder 3-Thienyl, 2- oder 3-Furyl und 1-Tetrazoyl, ist

3) einem Rest der allgemeinen Formel:

$$R_8OCO—$$

worin $R_8$ ein verzweigter, nicht substituierter Alkylrest, ein geradkettiger oder verzweigter Alkylrest, der einen oder mehrere Substituenten, ausgewählt aus den Halogenatomen oder den Cyano- und Phenylresten oder substituierten Phenylresten (durch ein oder mehrere Halogenatome oder Alkyl-, Alkyloxy-, Nitro- oder Phenylreste), trägt, ein 2-Trimethylsilyl-äthylrest, ein Vinyl- oder Allylrest oder ein Chinolylrest ist,

4) einem Rest der allgemeinen Formel:

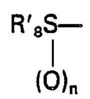

oder $R'_8Se—$

worin $R'_8$ einen Alkyl- oder Phenylrest oder substituierten Phenylrest (durch ein oder mehrere Halogenatome oder Nitro- oder Alkylreste) darstellt und n gleich 0 oder 1 ist,

5) oder $R_2NH—$ ist ersetzt durch einen Dimethylaminomethylenaminorest oder durch einen Rest der allgemeinen Formel:

$$Ar—CH{=}N—$$

worin Ar eine Phenylgruppe, gegebenenfalls substituiert durch einen oder mehrere Methoxy-, Nitro-, Alkyl- oder Hydroxyreste, ist, R'' ein Wasserstoffatom oder einen Methoxyrest in Position 7α oder ein Wasserstoffatom in Position 7β darstellt, und $R_3$ und $R_4$, die gleich oder verschieden sind, Alkylreste (gegebenenfalls substituiert durch einen Alkyloxy- oder Dialkylaminorest) oder Phenylreste darstellen oder zusammen mit dem Stickstoffatom, an das sie gebunden sind, einen gesättigten Heterocyclus mit 5 oder 6 Gliedern darstellen, der gegebenenfalls ein weiteres Heteroatom, ausgewählt aus Stickstoff, Sauerstoff oder Schwefel enthält und gegebenenfalls durch einen Alkylrest substituiert ist, wobei die oben erwähnten Alkylreste selbstverständlich geradkettig oder verzweigt sind und 1 bis 4 Kohlenstoffatome enthalten, dadurch gekennzeichnet, daß man eine Verbindung der allgemeinen Formel:

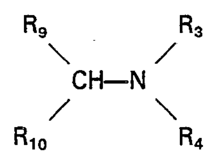

{worin $R_3$ und $R_4$ die obige Bedeutung haben und $R_9$ und $R_{10}$, die gleich oder voneinander verschieden sind, entweder Gruppen der allgemeinen Formel:

$$—X_2R_{11}$$

(in welcher $X_2$ ein Sauerstoffatom ist und $R_{11}$ einen Alkyl- oder Phenylrest darstellt) darstellen oder einer einen Rest $—X_2R_{11}$ (worin $X_2$ ein Sauerstoff- oder Schwefelatom ist und $R_{11}$ die obige Bedeutung hat) und der andere einen Aminorest der allgemeinen Formel

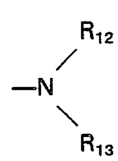

(in welcher $R_{12}$ und $R_{13}$ die Bedeutung von $R_3$ und $R_4$ haben) darstellt oder jeder einen Rest der allgemeinen Formel $—NR_{12}R_{13}$ darstellt} auf ein Oxacephalosporinderivat der allgemeinen Formel

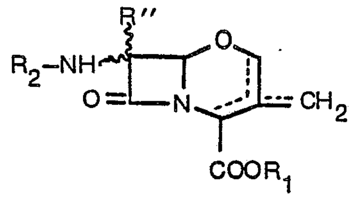

in welcher $R_1$, $R_2$ und R'' die oben angegebene Bedeutung haben und das in 2- oder 3-Bicycloocten-Form oder 3-Methylen-bicyclooctan-Form vorliegt, einwirken läßt und dann gegebenenfalls die erhaltene Verbindung in ihre Isomeren trennt.

**Claims for the contracting States: BE CH DE FR GB IT LI LU NL SE**

1. A novel oxacephalosporin derivative, characterised in that it corresponds to the general formula

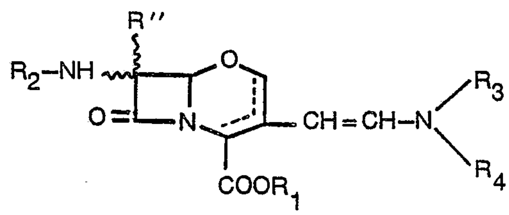

which is in the bicyclooct-2-ene or -3-ene form and in which the substituent on the carbon atom in the 3-position of the bicyclooctene has the E or Z stereoisomeric configuration, $R_1$ represents an easily removable acid-protecting radical, $R_2$ represents either a radical of the general formula:

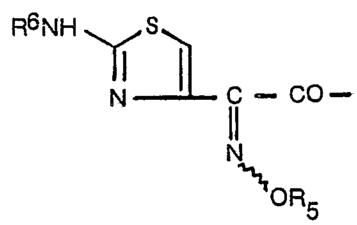

[in which $R_5$ is a hydrogen atom, an alkyl or vinyl radical, a carboxyalkyl radical of the general formula:

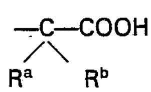

{in which $R^a$ and $R^b$ are identical or different and represent hydrogen atoms or alkyl radicals, or together form an alkylene ring containing 2 or 3 carbon atoms}, of which the acid group is protected, or an oxime-protecting radical, and $R^6$ is an amine-protecting radical], or an α-carboxyarylacetyl radical of which the acid group is protected and in which aryl represents phenyl, p-hydroxyphenyl, protected p-hydroxyphenyl or thien-2-yl or -3-yl, and R'' represents a hydrogen atom or a methoxy radical in the 7α-position, or $R_2$ represents an easily removable radical chosen from among:

1) benzhydryl or trityl,
2) an acyl radical of the general formula:

$R_7CO—$

in which $R_7$ represents:

a) a hydrogen atom, an alkyl radical containing 1 to 7 carbon atoms, methyl substituted by 1 to 3 halogen atoms, alkenyl containing 3 to 7 carbon atoms or cyanomethyl,

b) a phenyl radical (which can be substituted up to 3-fold by hydroxyl, nitro, cyano, trifluoromethyl, alkyl or alkoxy groups) or a thien-2-yl or -3-yl radical,

c) a radical of the general formula:

$$R'_7-Y-CH_2-$$

in which $R'_7$ is a radical as defined in b) and Y is a sulphur or oxygen atom, or

d) an arylalkyl radical of the general formula:

$$R''_7CH_2-$$

in which $R''_7$ is a phenyl radical which can be substituted up to 3-fold (by hydroxyl, alkyl or alkoxy radicals) or a heterocyclyl radical chosen from among thien-2-yl or -3-yl, fur-2-yl or -3-yl and tetrazol-1-yl,

3) a radical of the general formula:

$$R_8OCO-$$

in which $R_8$ is a branched unsubstituted alkyl radical, a straight or branched alkyl radical carrying one or more substituents chosen from among halogen atoms or cyano, phenyl or substituted phenyl radicals (the substituted phenyl radicals having one or more halogen atoms or alkyl, alkoxy, nitro or phenyl radicals as substituents), a 2-trimethylsilyl-ethyl radical, a vinyl or allyl radical or a quinolyl radical,

4) a radical of the general formula:

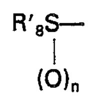 or $R'_8Se-$

in which $R'_8$ represents an alkyl, phenyl or substituted phenyl radical (the substituents of the substituted phenyl radical being one or more halogen atoms or nitro or alkyl radicals) and n is 0 or 1,

5) or $R_2NH-$ is replaced by a dimethylaminomethyleneamino radical or by a radical of the general formula:

$$Ar-CH=N-$$

in which Ar is a phenyl group which is optionally substituted by one or more methoxy, nitro, alkyl or hydroxyl radicals and R'' represents a hydrogen atom or a methoxy radical in the 7α-position or a hydrogen atom in the 7β-position and $R_3$ and $R_4$, which are identical or different, represent alkyl radicals (optionally substituted by an alkoxy or dialkylamino radical) or phenyl radicals, or form, together with the nitrogen atom to which they are attached, a saturated heterocyclic structure with 5 or 6 chain members, optionally containing another heteroatom chosen from among nitrogen, oxygen or sulphur and optionally substituted by an alkyl radical, it being understood that the alkyl radicals mentioned above are straight or branched and contain 1 to 4 carbon atoms.

2. A product according to Claim 1, characterised in that $R_1$ is an acid-protecting radical chosen from among methoxymethyl, t.-butyl, benzhydryl, benzyl, nitrobenzyl and p-methoxybenzyl.

3. Process for the preparation of a product according to Claim 1, characterised in that a product of the general formula:

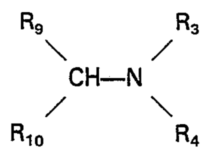

{in which $R_3$ and $R_4$ are defined as in Claim 1 and either $R_9$ and $R_{10}$, which are identical or different, represent groups of the general formula (in which $X_2$ is an oxygen atom and $R_{11}$ represents an alkyl or phenyl radical) or one of them represents a $-X_2R_{11}$ radical (in which $X_2$ is an oxygen or sulphur atom and $R_{11}$ is defined as above) and the other represents an amino radical of the general formula

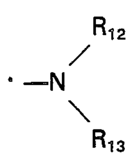

(in which $R_{12}$ and $R_{13}$ are defined like $R_3$ and $R_4$) or each of them represents a radical of the general formula $-NR_{12}R_{13}$}, is reacted with an oxacephalosporin derivative of the general formula:

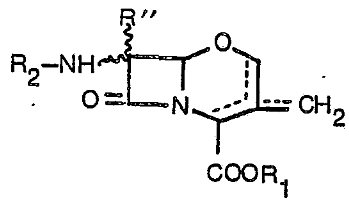

in which $R_1$, $R_2$ and R'' are defined as in Claim 1, and which is in the bicyclooct-2-ene or -3-ene or 3-methylene-bicyclooctane form, after which, if desired, the product obtained is separated into its isomers.

**Claim for the contracting State: AT**

Process for the preparation of a novel oxacephalosporin derivative of the general formula:

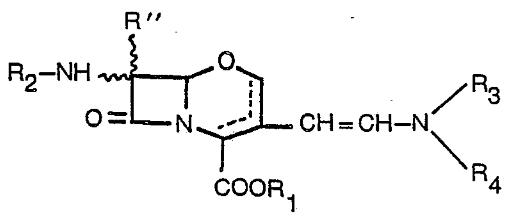

which is in the bicyclooct-2-ene or -3-ene form and in which the substituent on the carbon atom in the 3-position of the bicyclooctene has the E or Z stereoisomeric configuration, $R_1$ represents an easily removable acid-protecting radical, $R_2$ represents either a radical of the general formula:

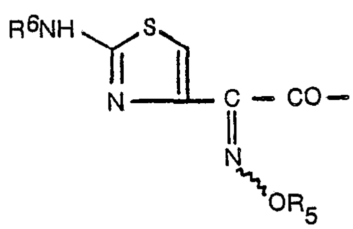

[in which $R_5$ is a hydrogen atom, an alkyl or vinyl radical, a carboxyalkyl radical of the general formula:

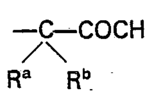

{in which $R^a$ and $R^b$ are identical or different and represent hydrogen atoms or alkyl radicals, or together form an alkylene ring containing 2 or 3 carbon atoms}, of which the acid group is protected, or an oxime-protecting radical, and $R^6$ is an amine-protecting radical], or an α-carboxyarylacetyl radical of which the acid group is protected and in which aryl represents phenyl, p-hydroxyphenyl, protected p-hydroxyphenyl or thien-2-yl or -3-yl, and R'' represents a hydrogen atom or a methoxy radical in the 7α-position, or $R_2$ represents an easily removable radical chosen from among:

1) benzhydryl or trityl,

2) an acyl radical of the general formula:

$$R_7CO-$$

in which $R_7$ represents:

a) a hydrogen atom, an alkyl radical containing 1 to 7 carbon atoms, methyl substituted by 1 to 3 halogen atoms, alkenyl containing 3 to 7 carbon atoms or cyanomethyl,

b) a phenyl radical (which can be substituted up to 3-fold by hydroxyl, nitro, cyano, trifluoromethyl, alkyl or alkoxy groups) or a thien-2-yl or -3-yl radical,

c) a radical of the general formula:

$$R'_7—Y—CH_2—$$

in which $R'_7$ is a radical as defined in b) and Y is a sulphur or oxygen atom, or

d) an arylalkyl radical of the general formula:

$$R''_7CH_2—$$

in which $R''_7$ as a phenyl radical which can be substituted up to 3-fold (by hydroxyl, alkyl or alkoxy radicals) or a heterocyclyl radical chosen from among thien-2-yl or -3-yl, fur-2-yl or -3-yl and tetrazol-1-yl,

3) a radical of the general formula:

$$R_8OCO—$$

in which $R_8$ is a branched unsubstituted alkyl radical, a straight or branched alkyl radical carrying one or more substituents chosen from among halogen atoms or cyano, phenyl or substituted phenyl radicals (the substituted phenyl radicals having one or more halogen atoms or allyl, alkoxy, nitro or phenyl radicals as substituents), a 2-trimethylsilyl-ethyl radical, a vinyl or allyl radical or a quinolyl radical,

4) a radical of the general formula:

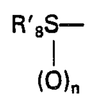 or $R'_8Se—$

in which $R'_8$ represents an alkyl, phenyl or substituted phenyl radical (the substituents of the substituted phenyl radical being one or more halogen atoms or nitro or alkyl radicals) and n is 0 or 1,

5) or $R_2NH—$ is replaced by a dimethylaminomethyleneamino radical or by a radical of the general formula:

$$Ar—CH=N—$$

in which Ar is a phenyl group which is optionally substituted by one or more methoxy, nitro, alkyl or hydroxyl radicals and R'' represents a hydrogen atom or a methoxy radical in the 7α-position or a hydrogen atom in the 7β-position and $R_3$ and $R_4$, which are identical or different, represent alkyl radicals (optionally substituted by an alkoxy or dialkylamino radical) or phenyl radicals, or form, together with the nitrogen atom to which they are attached, a saturated heterocyclic structure with 5 or 6 chain members, optionally containing another heteroatom chosen from among nitrogen, oxygen or sulphur and optionally substituted by an alkyl radical, it being understood that the alkyl radicals mentioned above are straight or branched and contain 1 to 4 carbon atoms, characterised in that a product of the general formula

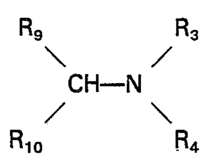

{in which $R_3$ and $R_4$ are defined as above and either $R_9$ and $R_{10}$, which are identical or different, represent groups of the general formula

$$—X_2R_{11}$$

(in which $X_2$ is an oxygen atom and $R_{11}$ represents an alkyl or phenyl radical) or one of them represents a $—X_2R_{11}$ radical (in which $X_2$ is an oxygen or sulphur atom and $R_{11}$ is defined as above) and the other represents an amino radical of the general formula

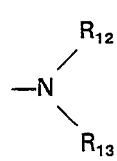

(in which $R_{12}$ and $R_{13}$ are defined like $R_3$ and $R_4$) or each of them represents a radical of the general formula —$NR_{12}R_{13}$}, is reacted with an oxacephalosporin derivative of the general formula:

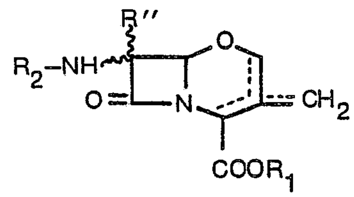

in which $R_1$, $R_2$ and R'' are defined as above, and which is in the bicyclooct-2-ene or -3-ene or 3-methylene-bicyclooctane form, after which, if desired, the product obtained is separated into its isomers.